(19) Europäisches Patentamt
European Patent Office
Office européen des brevets

(11) **EP 3 180 298 B1**

(12) **EUROPÄISCHE PATENTSCHRIFT**

(45) Veröffentlichungstag und Bekanntmachung des Hinweises auf die Patenterteilung:
**07.11.2018 Patentblatt 2018/45**

(21) Anmeldenummer: **15748049.2**

(22) Anmeldetag: **11.08.2015**

(51) Int Cl.:
*C07C 5/48* (2006.01)        *C07C 7/08* (2006.01)
*C07C 7/11* (2006.01)        *C07C 7/04* (2006.01)
*C07C 11/167* (2006.01)      *B01J 15/00* (2006.01)

(86) Internationale Anmeldenummer:
**PCT/EP2015/068436**

(87) Internationale Veröffentlichungsnummer:
**WO 2016/023892 (18.02.2016 Gazette 2016/07)**

(54) **VERFAHREN ZUR HERSTELLUNG VON 1,3-BUTADIEN AUS N-BUTENEN DURCH OXIDATIVE DEHYDRIERUNG**

METHOD FOR PRODUCING 1,3-BUTADIENE FROM N-BUTENES BY OXIDATIVE DEHYDROGENATION

PROCÉDÉ DE PRÉPARATION DE 1,3-BUTADIÈNE À PARTIR DE N-BUTÈNES PAR DÉSHYDROGÉNATION OXYDATIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.08.2014 EP 14180700**

(43) Veröffentlichungstag der Anmeldung:
**21.06.2017 Patentblatt 2017/25**

(73) Patentinhaber:
• **BASF SE**
**67056 Ludwigshafen am Rhein (DE)**
• **Linde AG**
**80331 München (DE)**

(72) Erfinder:
• **GRÜNE, Philipp**
**68161 Mannheim (DE)**
• **HAMMEN, Oliver**
**67587 Wintersheim (DE)**
• **ECKRICH, Rainer**
**67105 Schifferstadt (DE)**
• **JOSCH, Jan, Pablo**
**67434 Neustadt (DE)**

• **WALSDORFF, Christian**
**67061 Ludwigshafen (DE)**
• **BIEGNER, Andre**
**81479 München (DE)**
• **BLOCH, Gregor**
**82211 Herrsching (DE)**
• **BOELT, Heinz**
**82515 Wolfratshausen (DE)**
• **REYNEKE, Hendrik**
**81479 München (DE)**
• **TOEGEL, Christine**
**85579 Neubiberg (DE)**
• **WENNING, Ulrike**
**82049 Pullach (DE)**

(74) Vertreter: **Schuck, Alexander**
**Isenbruck Bösl Hörschler LLP**
**Patentanwälte**
**EASTSITE ONE**
**Seckenheimer Landstraße 4**
**68163 Mannheim (DE)**

(56) Entgegenhaltungen:
**WO-A1-2014/111406        US-A1- 2012 130 137**
**US-A1- 2014 200 381**

**Beschreibung**

Die Erfindung betrifft ein Verfahren zur Herstellung von 1,3-Butadien (Butadien) aus n-Butenen durch oxidative Dehydrierung (ODH).

Butadien ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (Acrylnitril-Butadien-StyrolCopolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, 1,3-Butadien und 1,2-Butadien, Butinen, Methylallen, $C_5$- und höheren Kohlenwasserstoffen an.

Butadien kann auch durch oxidative Dehydrierung von n-Butenen (1-Buten und/oder 2-Buten) erhalten werden. Als Einsatzgas für die oxidative Dehydrierung (Oxidehydrierung, ODH) von n-Butenen zu Butadien kann jedes beliebige n-Butene enthaltende Gemisch benutzt werden. Beispielsweise kann eine Fraktion verwendet werden, die als Hauptbestandteil n-Butene (1-Buten und/oder 2-Buten) enthält und aus der $C_4$-Fraktion eines Naphtha-Crackers durch Abtrennen von Butadien und Isobuten erhalten wurde. Des Weiteren können auch Gasgemische als Einsatzgas eingesetzt werden, die 1-Buten, cis-2-Buten, trans-2-Buten oder deren Gemische umfassen und durch Dimerisierung von Ethylen erhalten wurden. Ferner können als Einsatzgas n-Butene enthaltende Gasgemische eingesetzt werden, die durch katalytisches Wirbelschichtcracken (Fluid Catalytic Cracking, FCC) erhalten wurden.

Verfahren zur oxidativen Dehydrierung von Butenen zu Butadien sind grundsätzlich bekannt.

US 2012/0130137 A1 beispielsweise beschreibt ein solches Verfahren unter Verwendung von Katalysatoren, die Oxide von Molybdän, Bismuth und in der Regel weiteren Metallen enthalten. Für die nachhaltige Aktivität solcher Katalysatoren für die oxidative Dehydrierung ist ein kritisches Mindestmaß an Sauerstoffpartialdruck in der Gasatmosphäre erforderlich, um eine zu weitgehende Reduktion und damit einen Performance-Verlust der Katalysatoren zu vermeiden. Aus diesem Grund kann in der Regel auch nicht mit einem stöchiometrischen Sauerstoffeinsatz oder vollständigem Sauerstoff-Umsatz im Oxidehydrierungsreaktor (ODH-Reaktor) gearbeitet werden. In der US 2012/0130137 werden zum Beispiel ein Sauerstoffgehalt von 2,5 bis 8 Vol.-% im Ausgangsgas beschrieben.

Die Notwendigkeit eines Sauerstoffüberschusses für solche Katalysatorsysteme ist allgemein bekannt und schlägt sich in den Verfahrensbedingungen bei Verwendung derartiger Katalysatoren nieder. Stellvertretend seien die neueren Arbeiten von Jung et al. (Catal. Surv. Asia 2009, 13, 78-93; DOI 10.1007/s10563-009-9069-5 und Applied Catalysis A: General 2007, 317, 244-249; DOI 10.1016/j.apcata.2006.10.021) genannt.

In der JP-A 2011-006381 der Firma Mitsubishi wird das Risiko der Peroxidbildung im Aufarbeitungsteil eines Verfahrens zur Herstellung von konjugierten Alkadienen angesprochen. Zur Lösung wird der Zusatz von Polymerisationsinhibitoren zu den Absorptionslösungen für die Prozessgase und die Einstellung eines maximalen Peroxidgehalts von 100 Gewichts-ppm durch Erhitzen der Absorptionslösungen beschrieben. Allerdings werden keine Angaben zur Vermeidung oder Kontrolle von Peroxiden in vorgelagerten Verfahrensschritten gemacht. Kritisch ist insbesondere der Kühlschritt des ODH-Reaktoraustrags mit einem Wasser-Quench zu sehen. Gebildete organische Peroxide sind kaum in Wasser löslich, so dass sie sich abscheiden und in fester oder flüssiger Form im Apparat anreichern können, statt mit dem wässrigen Purge-Strom ausgetragen zu werden. Gleichzeitig ist die Temperatur des Wasser-Quenchs nicht so hoch, als dass von einem ausreichend hohen und stetigen Abbau der gebildeten Peroxide ausgegangen werden kann.

Bei der katalytischen oxidativen Dehydrierung können hochsiedende Nebenkomponenten wie beispielsweise Maleinsäureanhydrid, Phthalsäureanhydrid, Benzaldehyd, Benzoesäure, Ethylbenzol, Styrol, Fluorenon, Anthrachinon und andere gebildet werden. Ablagerungen dieser Komponenten können zu Verstopfungen und zu einem Druckverlustanstieg im Reaktor oder hinter dem Reaktor im Bereich der Aufarbeitung führen und so einen geregelten Betrieb stören. Ablagerungen der genannten hochsiedenden Nebenkomponenten können auch die Funktion von Wärmetauschern beeinträchtigen oder bewegte Apparate wie Kompressoren schädigen. Wasserdampfflüchtige Verbindungen wie Fluorenon können durch einen mit Wasser betriebenen Quench-Apparat durchschlagen und sich dahinter in den Gasaustragsleitungen niederschlagen. Damit besteht grundsätzlich auch die Gefahr, dass feste Ablagerungen in nachgeschaltete Apparateteile, wie beispielsweise Kompressoren, gelangen und dort Schaden anrichten.

In der US 2012/0130137 A1 Absatz [0122] wird auch auf die Problematik hochsiedender Nebenprodukte hingewiesen. Insbesondere werden Phthalsäureanhydrid, Anthrachinon und Fluorenon genannt, die typischerweise in Konzentrationen von 0,001 bis 0,10 Vol.-% im Produktgas vorlägen. In der US 2012/0130137 A1 Absatz [0124] - [0126] wird empfohlen, die heißen Reaktoraustragsgase direkt durch Kontakt mit einer Kühlflüssigkeit (Quench-Turm) auf üblicherweise zunächst 5 bis 100°C abzukühlen. Als Kühlflüssigkeiten werden Wasser oder wässrige AlkaliLösungen genannt. Ausdrücklich wird die Problematik von Verstopfungen im Quench durch Hochsieder aus dem Produktgas oder durch Polymerisationsprodukte hochsiedender Nebenprodukte aus dem Produktgas erwähnt, weshalb es vorteilhaft sei, dass hochsiedende Nebenprodukte so wenig wie möglich aus dem Reaktionsteil in den Kühlungsteil (Quench) mitgetragen werden.

In JP-A 2011-001341 wird für ein Verfahren zur oxidativen Dehydrierung von Alkenen zu konjugierten Alkadienen eine zweistufige Kühlung beschrieben. Dabei wird das Produktaustragsgas der oxidativen Dehydrierung zunächst auf eine Temperatur zwischen 300 und 221°C abgekühlt und dann auf eine Temperatur zwischen 99 und 21°C weiter abgekühlt. In den Absätzen [0066] ff wird beschrieben, dass zur Einstellung der Temperatur zwischen 300 und 221°C bevorzugt Wärmetauscher eingesetzt werden, wobei aber auch ein Teil der Hochsieder aus dem Produktgas in diesen Wärmetauschern ausfallen könnten. In der JP-A 2011-001341 wird deshalb ein gelegentliches Auswaschen von Ablagerungen aus den Wärmetauschern mit organischen oder wässrigen Lösungsmitteln beschrieben. Als Lösungsmittel werden beispielsweise aromatische Kohlenwasserstoffe wie Toluol oder Xylol oder ein alkalisches wässriges Lösungsmittel wie beispielsweise die wässrige Lösung von Natriumhydroxid beschrieben. Um eine zu häufige Unterbrechung des Verfahrens zur Reinigung des Wärmetauscher zu vermeiden, wird in der der JP-A 2011-001341 ein Aufbau mit zwei parallel angeordneten Wärmetauschern beschrieben, die jeweils abwechselnd betrieben oder gespült werden (sogenannte A/B-Fahrweise).

JP-A 2013-119530 beschreibt einen Quench, in dem ein ODH-Produktgas durch direkten Kontakt mit Wasser gekühlt wird. In Absatz 7 wird auf das Problem eingegangen, dass das Produktgas feste Bestandteile mit sich führt und dass diese einen stabilen Betrieb verhindern können. Feste Bestandteile würden sogar im Abgas der Quenchkolonne gefunden. In Absatz 41 wird behauptet, dass diese Bestandteile zu einem großen Teil aus Isophthalsäure und Terephthalsäure bestehen. Auch wenn die Menge im Abgas gering sei, könnten z. B. Filter sehr schnell belegt werden. Gemäß dieser Anmeldung werden die festen Bestandteile durch eine geeignete Wahl der Einbauten und des Volumenstromverhältnis von Kühlmittel und Gasstrom so gut es geht aus dem Produktgas beseitigt. Die Anmeldung macht jedoch keine Angaben, wie eine Blockierung des Kühlmittelumlaufs verhindert werden kann.

JP-A 2013-177380 beschreibt in Absatz 60 mögliche im Produktgas-Quench eingesetzte Kühlmittel. Als Kühlflüssigkeit werden allgemein gesättigte Kohlenwasserstoffe, ungesättigte Kohlenwasserstoffe, aromatische Kohlenwasserstoffe, Ester, Ether, Aldehyde, Ketone, Amine, Säuren, Wasser sowie Gemische davon genannt. Bevorzugtes Kühlmittel ist Wasser. In Absatz 62

### Geänderte Beschreibungsseiten - Reinschrift

wird das Zu- und Abführen von Wasser als Kühlmittel beschrieben: Demgemäß kann zumindest ein Teil des Wassers, das aus dem Sumpf des Kühlturms abgelassen wurde, einer mittleren Stufe und/oder dem Kopf des Kühlturms wieder zugeführt werden. Das dem Sumpf entnommene Wasser kann Feststoffe enthalten. Zu deren Abtrennung schlägt die Schrift gängige Verfahren, beispielsweise die Verwendung eines Siebs, vor. In den Absätzen 63 und 64 werden als im Kühlmittel auskondensierende Nebenprodukte sauerstoffhaltige organische Verbindungen, wie Aldehyde, Ketone, Carbonsäure, ungesättigte Aldehyde, ungesättigte Carbonsäure sowie Polymere, welche die genannten Verbindungen als Struktureinheit aufweisen, genannt. Die Schrift macht keine Aussage darüber, wie trotz des Feststoffgehaltes ein stabiler Umlauf des Kühlmittels gewährleistet werden kann.

Gemäß WO 2012/157495 wird im Produktgas-Quench einer Oxidehydrierung als Kühlmittel die wässrige Lösung eines organischen Amins eingesetzt. In Absatz 6 wird das Problem der Verstopfung von Leitungen durch Feststoffe beschrieben. Dementsprechend wurde festgestellt, dass hochsiedenden Nebenprodukte, wie organische Säuren, Aldehyde und Ketone, bei der Abschreckung des Reaktionsproduktgases mit Kühlwassers kondensieren und mit dem Fluss des Reaktionsproduktgases mitfließen, wodurch Leitungen verstopft und der kontinuierliche Betrieb der Anlage gefährdet wird.

Eine effektive Abtrennung der Komponenten wird demgemäß durch Verwendung einer wässrigen Lösung eines organischen Amins sowie eines bevorzugt aromatischen Lösemittels erreicht. Die beiden Kühlmittel werden aber in getrennten Bereichen des Kühlturms eingesetzt. So heißt es in Absatz 35, dass für das Waschen des Reaktionsproduktgases mit der wässrigen Lösung von organischem Amin ein erster Abschreck-Turm und für die Reinigung des Reaktionsproduktgases mit dem aromatischen Lösungsmittel ein zweiter Abschreck-Turm verwendet werden.

KR 2013-0036467 und KR 2013-0036468 beschreiben die Verwendung eines Gemischs aus Wasser und einem mit Wasser mischbaren organischen Lösemittel als Kühlmittel in einem Produktgasquench einer Oxidehydrierung. Wegen der Wassermischbarkeit ist die Aufarbeitung und Regenerierung des organischen Lösemittels sehr energieintensiv und unter wirtschaftlichen Gesichtspunkten unvorteilhaft.

US 2014/200381 A1 offenbart ein Verfahren zur Herstellung von Butadien aus n-Butenen umfassend eine oxidative Dehydrierung, einen Abkühlschritt mit einem im Kreis geführten Kühlmittel, einen Kompressionsschritt, eine Abtrennung von Gasbestandteilen durch Absorption und Desorption sowie die Abtrennung von Butadien durch Extraktivdestillation und Destillation des Extraktivlösungsmittels. Als Kühlmittel werden Wasser, alkalische wässrige Lösungen, organische Lösungsmittel oder Mischungen davon offenbart. Bevorzugtes Kühlmedium ist Toluol.

WO 2014/111406 A1 offenbart ebenfalls ein Verfahren zur Herstellung von Butadien aus n-Butenen umfassend eine oxidative Dehydrierungsreaktion, einen Abkühlschritt mit einem im Kreislauf geführten Kühlmittel, einen Kompressionsschritt, eine Abtrennung von Gasbestandteilen durch Absorption und Desorption sowie die Abtrennung von Butadien durch Extraktivdestillation und Destillation des Extraktivlösungsmittels. Als Kühlmedium wird Wasser verwendet.

Aufgabe der vorliegenden Erfindung ist es, ein Verfahren bereitzustellen, das den oben genannten Nachteilen bekannter Verfahren abhilft. Insbesondere soll ein Verfahren bereitgestellt werden, bei dem Ablagerungen durch hochsiedende organische Nebenbestandteile in den der ODH nachgeschalteten Apparaten vermieden werden. Weiterhin soll ein Verfahren bereitgestellt werden, bei dem die mögliche Anreicherung organischer Peroxide vermieden wird. Insbesondere soll ein Verfahren bereitgestellt werden, bei dem es im Kühlmittelumlauf (Quenchumlauf), insbesondere in den Düsen, durch die das Kühlmittel in die Abkühlzone eingespeist wird, nicht zu Verstopfungen durch im Kühlmittel dispergierte Feststoffe kommt, und ein stabiler kontinuierlicher Quenchumlauf gewährleistet ist.

Gelöst wird die Aufgabe durch ein Verfahren zur Herstellung von Butadien aus n-Butenen mit den Schritten:

A) Bereitstellung eines n-Butene enthaltenden Einsatzgasstroms a;

B) Einspeisung des n-Butene enthaltenden Einsatzgasstromes a und mindestens eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird;

Ca) Abkühlung des Produktgasstroms b durch Inkontaktbringen mit einem Kühlmedium in mindestens einer Abkühlzone, wobei das Kühlmedium zumindest teilweise zurückgeführt wird und eine wässrige und eine organische Phase aus einem organischen Lösungsmittel aufweist, und das Massenverhältnis der wässrigen Phase zur organischen Phase in dem Kühlmedium beim Einspeisen in die Abkühlzonen vor dem Inkontaktbringen mit dem Produktgasstrom von 0,15 : 1 bis 10 : 1 beträgt;

Cb) Kompression des abgekühlten und gegebenenfalls an hochsiedenden Nebenkomponenten abgereicherten Produktgasstroms b in mindestens einer Kompressionsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;

D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der $C_4$-Kohlenwasserstoffe umfassend Butadien und n-Butene in einem Absorptionsmittel, wobei ein mit $C_4$-Kohlenwasserstoffen beladener Absorptionsmittelstrom und der Gasstrom d2 erhalten werden, und anschließende Desorption der $C_4$-Kohlenwasserstoffe aus dem beladenen Absorptionsmittelstrom, wobei ein $C_4$-Produktgasstrom d1 erhalten wird;

E) Auftrennung des $C_4$-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;

F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

Es wurde gefunden, dass ein kontinuierlicher Betrieb des Quenchumlaufes länger möglich ist, wenn der Umlauf mit zwei miteinander nicht mischbaren Kühlmitteln betrieben wird. Weiterhin ist ein kontinuierlicher Betrieb besonders lange möglich, wenn die beiden nicht mischbaren Lösemittel beim Eintritt in die Quenchkolonne in einem bestimmten Verhältnis stehen. Weiterhin ist ein kontinuierlicher Betrieb besonders lang möglich, wenn die beiden nicht mischbaren Lösemittel beim Eintritt in die Quenchkolonne innig miteinander dispergiert sind.

Erfindungsgemäß wird in der Abkühlstufe Ca) eine zweiphasige Dispersion aus einem oder mehreren organischen Lösungsmitteln und einer wässrigen Phase verwendet. Durch die rasche Abkühlung des Produktgasstroms im Quench kommt es zur Kondensation von hochsiedenden Nebenkomponenten. Organische Lösungsmittel weisen im Allgemeinen ein sehr viel höheres Lösungsvermögen für die hochsiedende Nebenprodukte, die in den dem ODH-Reaktor nachgelagerten Anlageteilen zu Ablagerungen und Verstopfungen führen können, als Wasser oder alkalisch-wässrige Lösungen auf. Erfindungsgemäß eingesetzte organische Lösungsmittel sind aromatische Kohlenwasserstoffe, besonders bevorzugt sind Toluol, o-Xylol, m-Xylol, p-Xylol, Mesitylen, Mono-, Di- und Triethylbenzol, Mono-, Di- und Triisopropylbenzol sowie Gemische daraus.

Ein ganz besonders bevorzugt eingesetztes Lösungsmittel ist Mesitylen. Im Allgemeinen liegen diese Lösungsmittel in einer Reinheit von mindestens 90 Gew.-% vor.

Im technischen Maßstab sind organische Lösungsmittel oft nicht als reine Substanzen erhältlich. Ein organisches Lösungsmittel besitzt normalerweise eine Reinheit von mindestens 90 Gew.-%, bevorzugt von mindestens 94 Gew.-% und besonders bevorzugt von mindestens 96 Gew.-%.

Beispielsweise enthält technisches Mesitylen im Allgemeinen mindestens 90 Gew.-% reines Mesitylen, bevorzugt mindestens 94 Gew.-% Mesitylen und besonders bevorzugt mindestens 96 Gew.-% Mesitylen. Typische Verunreinigungen umfassen andere Isomere von Trimethylbenzol, Isomere von Dimethylbenzol und Monomethylbenzol, sowie Isomere von Mono-, Di- und Triethylbenzol, Mono-, Di- und Triisopropylbenzol, aber auch kleinere Mengen weiterer aromatischer, insbesondere alkylaromatischer Verbindungen. Ebenso können geringe Mengen von aliphatischen Nebenkomponenten enthalten sein

Es wurde zudem gefunden, dass durch die Gegenwart einer zusätzlichen wässrigen Phase in dem umlaufenden Kühlmedium Verstopfungen im Quenchumlauf, insbesondere im Bereich der Düsen, durch die das Kühlmittel die Quenchkolonne betritt, aber auch beispielsweise in den Pumpen des Kühlmittelumlaufs und in Messgeräten, welche den Volumenstrom des Umlaufs messen, wirksam vermieden werden können. Dies wird darauf zurückgeführt, dass es unter den kondensierten, hochsiedenden Nebenkomponenten auch Substanzen gibt, welche in einem organischen Lösemittel nur eine geringe Löslichkeit besitzen, sich in Wasser oder wässrigen Lösungen aber wesentlich besser lösen. Dies führt dazu, dass verklebend wirkende Substanzen in der organischen und wässrigen Phase gelöst werden, wodurch koksartige, unlösliche Feststoffe im Kühlmittelumlauf dispergiert bleiben, und sich nicht an Anlagenteilen wie Düsen ablagern und dort zu Verstopfungen führen.

Das Phasenverhältnis, d. h. das Verhältnis der Masse der wässrigen Phase zur Masse der organischen Phase des Kühlmediums beim Eintritt in die Abkühlstufe (Quenchstufe) vor dem Inkontaktbringen, wird bestimmt durch die Mengenströme der dem Kühlmittelumlauf zugegebenen wässrigen und organischen Kühlmittel, dem Mengenstrom an Wasserdampf, der im Produktgasstrom enthalten ist, den Mengenströmen an Wasserdampf und organischem Kühlmittel, welche die Abkühlstufe verlassen, und den Mengenströmen der wässrigen und organischen Phase, die dem Kühlmittelumlauf als Abstrom (Purge) entnommen werden. Das Phasenverhältnis ist größer oder gleich 0,13 : 1, bevorzugt größer oder gleich 0,15 : 1, besonders bevorzugt größer oder gleich 0,18 : 1 und insbesondere größer oder gleich 0,2 : 1 und speziell größer oder gleich 0,3 : 1 und kleiner oder gleich 100 : 1, bevorzugt kleiner oder gleich 10 : 1, besonders bevorzugt kleiner oder gleich 2 : 1, insbesondere kleiner oder gleich 1 : 1. Bevorzugte Bereiche des Phasenverhältnisses sind 0,15 : 1 bis 10 : 1, insbesondere 0,18 : 1 bis 2 : 1.

Vorzugsweise weist das Kühlmedium beim Eintritt in die Abkühlzone eine sehr gute Dispersion beider Phasen auf. Als Maß für die Dispersionsgüte wird ein relative Standardabweichung $\sigma/\sigma 0$ zugrunde gelegt. Siehe beispielsweise Kraume et al., "Continuous Mixing of Fluids" in Ull-mann's Encyclopedia of Industrial Chemistry, Wiley-VCH (2012); oder Streiff, Chem. Ing. Tech. 52, 520 (1980). Als Messmethode wird die Leitfähigkeitsmessmethode nach Phal und Muschelknautz, Chem. Ing. Tech. 51, 347 (1979), verwendet. Hierbei werden die unterschiedlichen elektrischen Leitfähigkeiten von wässriger und organischer Phase ausgenutzt und die elektrische Leitfähigkeit und damit die Konzentration der beiden Phasen ortsaufgelöst vermessen. Eine ideale Dispersion würde also bei $\sigma/\sigma 0 = 0$ vorliegen. Bevorzugt weisen die die organische Phase bildenden Komponenten und die die wässrige Phase bildenden Komponenten des zweiphasigen Kühlmediums einen Variationskoeffizienten von kleiner 1, bevorzugt von kleiner 0,5, besonders bevorzugt von kleiner 0,1 auf.

Ein hoher Grad der Dispersion des Kühlmediums kann beispielsweise durch den Einbau geeigneter Mischer in den Umlauf erfolgen. Die Art der Mischer ist hier nicht weiter eingeschränkt und umfasst Rührer, statische Mischer und Blenden.

Weiterhin kann ein hoher Grad der Dispersion des Kühlmediums mittels einer Düse erzielt werden. Für die Reynoldszahl Re einer in einer Düse erzeugten Strömung wird für jede der zwei Phasen des Kühlmittels angenommen:

$$Re = (\square \times v \times d)/\square\square \text{ mit}$$

$\square$ = Dichte der jeweiligen Phase
$v$ = Strömungsgeschwindigkeit der jeweiligen Phase
$d$ = Länge (hier Öffnung der Düse)
$\square$ = dynamische Viskosität der jeweiligen Phase

Für Wasser mit einem Umlaufstrom von 60 1/h, einer Öffnung der Düse von 1,15 mm und einer dynamischen Viskosität von Wasser bei 20 °C von $10^{-3}$ Pa s ergibt sich beispielsweise:

$$v = \text{Volumenstrom / Fläche} = 1{,}66 \ 10^{-5} / (pi * (1{,}15/2 \ 10^{-3})^2) = 16 \text{ m/s}$$

$$Re = (1000\ kg/m^3\ x\ 16\ m/s\ x\ 1{,}15\ 10^{-3}\ m)/\ 10^{-3}\ Pa\ s = 18400.$$

Im Allgemeinen ist die Reynoldszahl Re der beiden Phasen des Kühlmediums bei Eintritt in die Abkühlstufe größer als 100, bevorzugt größer als 500, und besonders bevorzugt größer als 1000.

Entscheidend für einen hohen Grad der Dispersion ist weiterhin ein hoher volumenspezifischer Leistungseintrag in das Kühlmedium. Dieser kann wiederum beispielsweise durch geeignete Mischer, Pumpen oder Düsen erreicht werden.

Der volumenspezifische Leistungseintrag $P_v$ wird angenommen als

$$P_v = \Delta p \dot{V}/(V) \text{ mit}$$

$\Delta p$ = Druckverlust über das leistungseintragende Prozesseinheit

$\dot{V}$ = Umlaufvolumenstrom des Kühlmittels

$V$ = spezifische Volumen der Prozesseinheit

Für ein Kühlmedium mit einem Umlaufvolumenstrom von 60 1/h, einem Druckverlust über die Düse von 500 mbar und einem Volumen der Düse von 0,1 cm$^3$ ergibt sich beispielsweise:

$$P_v = 500\ mbar\ x\ 60\ l/h\ /\ 10\ mm^3 = 5\ 10^4\ (kg/ms^2)\ x\ 1.6\ 10^{-5}\ (m^3/s)\ /\ 10^{-7}\ m^3 = 8\ 10^7\ W/m^3.$$

Im Allgemeinen ist der volumenspezifische Leistungseintrag in das Kühlmittel im Umlauf mindestens $10^3\ W/m^3$, bevorzugt mindestens $10^4\ W/m^3$, und besonders bevorzugt mindestens $10^5\ W/m^3$.

Im Allgemeinen wird das Kühlmedium durch eine oder mehrere Düsen in die Abkühlzone oder die Abkühlzonen eingespeist. In einer bevorzugten Ausführungsform wird dabei in der oder den

Düsen eine Strömung mit einer Reynoldszahl Re von mindestens 1000 erzeugt. Der Leistungseintrag beträgt dabei mindestens $10^3\ W/m^3$. Insbesondere wird dadurch eine so gute Dispersion beider Phasen erzielt, dass der Variationskoeffizient jeder Komponente jeder Phase des Kühlmediums beim Eintritt in die Abkühlzonen kleiner 1 beträgt.

Nachstehende Ausführungsformen sind bevorzugte bzw. besonders bevorzugte Varianten des erfindungsgemäßen Verfahrens:

Die Stufe Ca) wird mehrstufig in Stufen Ca1) bis Can), bevorzugt zweistufig in zwei Stufen Ca1) und Ca2) durchgeführt. Dabei kann zumindest ein Teil des Kühlmediums nach Durchlaufen der zweiten Stufe Ca2) als Abkühlmittel der ersten Stufe Ca1) zugeführt werden.

Die Stufe Cb) umfasst im Allgemeinen mindestens eine Kompressionsstufe Cba) und mindestens eine Abkühlstufe Cbb). Bevorzugt wird in der mindestens einen Abkühlstufe Cbb) das in der Kompressionsstufe Cba) komprimierte Gas mit einem Abkühlmittel in Kontakt gebracht. Besonders bevorzugt enthält das Abkühlmittel der Abkühlstufe Cbb) das gleiche organische Lösungsmittel, das in der Stufe Ca) als Abkühlmittel verwendet wird. In einer insbesonders bevorzugten Variante wird zumindest ein Teil dieses Abkühlmittels nach Durchlaufen der mindestens einen Abkühlstufe Cbb) als Abkühlmittel der Stufe Ca) zugeführt.

Bevorzugt umfasst die Stufe Cb) mehrere Kompressionsstufen Cba1) bis Cban) und Abkühlstufen Cbb1) bis Cbbn), beispielsweise vier Kompressionsstufen Cba1) bis Cba4) und vier Abkühlstufen Cbb1) bis Cbb4).

Bevorzugt umfasst Schritt D) die Schritte Da) bis Dc):

Da) Absorption der $C_4$-Kohlenwasserstoffe umfassend Butadien und n-Butene in einem hochsiedenden Absorptionsmittel, wobei ein mit $C_4$-Kohlenwasserstoffen beladener Absorptionsmittelstrom und der Gasstrom d2 erhalten werden,

Db) Entfernung von Sauerstoff aus dem mit $C_4$-Kohlenwasserstoffen beladenen Absorptionsmittelstrom aus Schritt Da) durch Strippung mit einem nicht kondensierbaren Gasstrom, und

Dc) Desorption der $C_4$-Kohlenwasserstoffe aus dem beladenen Absorptionsmittelstrom, wobei ein $C_4$-Produktgasstrom d1 erhalten wird, der im Wesentlichen aus $C_4$-Kohlenwasserstoffen besteht und weniger als 100 ppm Sauerstoff umfasst.

Bevorzugt ist das in Schritt Da) eingesetzte hochsiedende Absorptionsmittel ein aromatisches Kohlenwasserstofflösungsmittel, besonders bevorzugt das in Schritt Ca) eingesetzte aromatische Kohlenwasserstofflösungsmittel, insbesondere Toluol, o-Xylol, m-Xylol, p-Xylol, Mesitylen oder Gemische daraus.

In einem Schritt A) wird ein n-Butene enthaltender Einsatzgasstrom bereitgestellt.

Als Einsatzgasstrom können reine n-Butene (1-Buten und/oder cis-/trans-2-Buten), aber auch Butene enthaltende Gasgemische eingesetzt werden. Ein solches Gasgemisch kann beispielsweise durch nicht-oxidative Dehydrierung von n-Butan erhalten werden. Es kann auch eine Fraktion eingesetzt werden, die als Hauptbestandteil n-Butene (1-Buten und cis-/trans-2-Buten) enthält und aus der $C_4$-Fraktion des Naphtha-Crackens durch Abtrennung von Butadien und iso-Buten erhalten wurde. Des Weiteren können auch Gasgemische als Einsatzgas eingesetzt werden, die reines 1-Buten, cis-2-Buten, trans-2-Buten oder Mischungen daraus umfassen, und die durch Dimerisierung von Ethylen erhalten wurden. Ferner können als Einsatzgas n-Butene enthaltende Gasgemische eingesetzt werden, die durch katalytisches Wirbelschichtkracken (Fluid Catalytic Cracking, FCC) erhalten wurden.

In einer Ausführungsform des erfindungsgemäßen Verfahrens wird das n-Butene enthaltende Einsatzgas durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien, 1-Buten, 2-Butene und nicht umgesetztem n-Butan, Nebenbestandteile enthält. Übliche Nebenbestandteile sind Wasserstoff, Wasserdampf, Stickstoff, CO und $CO_2$, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

In Schritt B) werden der n-Butene enthaltende Einsatzgasstrom und mindestens ein sauerstoffhaltiges Gas in mindestens eine Dehydrierzone eingespeist und die in dem Gasgemisch enthaltenen Butene in Gegenwart eines Oxidehydrierungskatalysators oxidativ zu Butadien dehydriert.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 ($Mo_{12}BiFe_{0,1}Ni_8ZrCr_3K_{0,2}O_x$ und $Mo_{12}BiFe_{0,1}Ni_8AlCr_3K_{0,2}O_x$), US 4,424,141 ($Mo_{12}BiFe_3Co_{4,5}Ni_{2,5}P_{0,5}K_{0,1}O_x$ + $SiO_2$), DE-A 25 30 959 ($Mo_{12}BiFe_3Co_{4,5}Ni_{2,5}Cr_{0,5}K_{0,1}O_x$, $Mo_{13,75}BiFe_3Co_{4,5}Ni_{2,5}Ge_{0,5}K_{0,8}O_x$, $Mo_{12}BiFe_3Co_{4,5}Ni_{2,5}Mn_{0,5}K_{0,1}O_x$ und $Mo_{12}BiFe_3Co_{4,5}Ni_{2,5}La_{0,5}K_{0,1}O_x$), US 3,911,039 ($Mo_{12}BiFe_3Co_{4,5}Ni_{2,5}Sn_{0,5}Ko_{0,1}O_x$), DE-A 25 30 959 und DE-A 24 47 825 ($Mo_{12}BiFe_3Co_{4,5}Ni_{2,5}W_{0,5}K_{0,1}O_x$).

Geeignete Multimetalloxide und deren Herstellung sind weiterhin beschrieben in US 4,423,281 ($Mo_{12}BiNi_8Pb_{0,5}Cr_3K_{0,2}O_x$ und $Mo_{12}Bi_bNi_7Al_3Cr_{0,5}K_{0,5}O_x$), US 4,336,409 ($Mo_{12}BiNi_6Cd_2Cr_3P_{0,5}O_x$), DE-A 26 00 128 ($Mo_{12}BiNi_{0,5}Cr_3P_{0,5}Mg_{7,5}K_{0,1}O_x$ + $SiO_2$) und DE-A 24 40 329 ($Mo_{12}BiCo_{4,5}Ni_{2,5}Cr_3P_{0,5}K_{0,1}O_x$).

Besonders bevorzugte katalytisch aktive, Molybdän und mindestens ein weiteres Metall enthaltende Multimetalloxide weisen die allgemeine Formel (Ia) auf:

$$Mo_{12}Bi_aFe_bCo_cNi_dCr_eX^1_fX^2_gO_y \qquad (Ia),$$

mit

$X^1$ = Si, Mn und/oder Al,
$X^2$ = Li, Na, K, Cs und/oder Rb,
$0,2 \leq a \leq 1$,
$0,5 \leq b \leq 10$,
$0 \leq c \leq 10$,
$0 \leq d \leq 10$,
$2 \leq c+d \leq 10$
$0 \leq e \leq 2$,
$0 \leq f \leq 10$
$0 \leq g \leq 0,5$
$y$ = eine Zahl, die unter der Voraussetzung der Ladungsneutralität durch die Wertigkeit und Häufigkeit der von Sauerstoff verschiedenen Elemente in (Ia) bestimmt wird.

Bevorzugt sind Katalysatoren, deren katalytisch aktive Oxidmasse von den beiden Metallen Co und Ni nur Co aufweist (d = 0). Bevorzugte ist $X^1$ Si und/oder Mn und $X^2$ ist vorzugsweise K, Na und/oder Cs, besonders bevorzugt ist $X^2$ = K.

Das molekularen Sauerstoff enthaltende Gas enthält im Allgemeinen mehr als 10 Vol.-%, vorzugsweise mehr als 15 Vol.-% und noch mehr bevorzugt mehr als 20 Vol.-% molekularen Sauerstoff. Bevorzugt ist es Luft. Die Obergrenze für den Gehalt an molekularem Sauerstoff beträgt im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, $CO_2$ und Wasser genannt werden. Die Menge an Inertgasen beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger.

Zur Durchführung der oxidativen Dehydrierung bei Vollumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann das Einsatzgas mit Sauerstoff oder einem oder mehreren sauerstoffhaltigen Gasen, beispielsweise Luft, und gegebenenfalls zusätzlichem Inertgas oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490°C, bevorzugt zwischen 300 bis 450°C und besonders bevorzugt zwischen 350 und 420°C.

Auf Grund der Exothermie der ablaufenden Reaktionen kann die Temperatur in bestimmten Abschnitten des Reaktorinneren während der Reaktion höher liegen als diejenige des Wärmeaustauschmittels und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1 bis 150°C, bevorzugt zwischen 10 bis 100°C und besonders bevorzugt zwischen 20 bis 80°C. Die Temperatur am Ende des Katalysatorbettes liegt im Allgemeinen zwischen 0 bis 100°C, vorzugsweise zwischen 0,1 bis 50°C, besonders bevorzugt zwischen 1 bis 25°C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

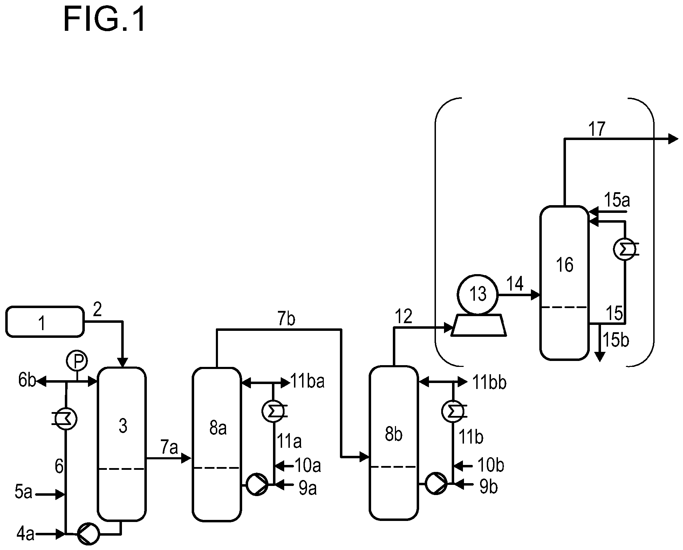

Die Erfindung wird nachstehend unter Bezugnahme auf die Figuren 1 bis 4 näher erläutert.

Die Katalysatorschüttung, die im ODH-Reaktor 1 eingebaut ist, kann aus einer einzelnen Schicht oder aus 2 oder einer Folge variabler Schichten bestehen (eine so genannte strukturierte Schüttung). Diese Schichten können aus reinem Katalysator bestehen oder mit einem Material verdünnt sein, das nicht mit dem Einsatzgas oder Komponenten aus dem Produktgas der Reaktion reagiert. Weiterhin können die Katalysatorschichten aus Formkörpern aus Vollmaterial oder geträgerten Schalenkatalysatoren bestehen.

Der die oxidative Dehydrierung verlassende Produktgasstrom 2 enthält neben Butadien im Allgemeinen noch nicht umgesetztes 1-Buten und 2-Buten, Sauerstoff sowie Wasserdampf. Als Nebenkomponenten enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate. Oxygenate können beispielsweise Formaldehyd, Furan, Acetaldehyd, Essigsäure, Maleinsäureanhydrid, Ameisensäure, Methacrolein, Acrolein, Propionaldehyd, Methacrylsäure, Crotonaldehyd, Crotonsäure, Propionsäure, Acrylsäure, Methylvinylketon, Styrol, Benzaldehyd, Benzoesäure, Phthalsäureanhydrid, Fluorenon, Anthrachinon und Butyraldehyd sein.

Der Produktgasstrom 2 am Reaktorausgang ist durch eine Temperatur nahe der Temperatur am Ende des Katalysatorbetts charakterisiert. Der Produktgasstrom wird dann auf eine Temperatur von 150 bis 400 °C, bevorzugt 160 bis 300 °C, besonders bevorzugt 170 bis 250 °C gebracht. Es ist möglich, die Leitung, durch die der Produktgasstrom fließt, um die Temperatur im gewünschten Bereich zu halten, zu isolieren oder einen Wärmetauscher einzusetzen.

Dieses Wärmetauschersystem ist beliebig, solange mit diesem System die Temperatur des Produktgases auf dem gewünschten Niveau gehalten werden kann. Als Beispiel eines Wärmetauschers können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes organisches Lösungsmittel kann ein Lösungsmittel, solange es in der Lage ist, die hochsiedenden Nebenprodukte aufzulösen, verwendet werden. Beispiele sind aromatische Kohlenwasserstofflösungsmittel, wie z. B. Toluol und Xylole und Mesitylen, sowie alkalische wässrige Lösungsmittel, wie z. B. die wässrige Lösung von Natriumhydroxid.

Anschließend wird aus dem Produktgasstrom 2 durch Abkühlung und Kompression ein Großteil der hochsiedenden Nebenkomponenten und des Wassers abgetrennt werden. Die Abkühlung erfolgt erfindungsgemäß durch Inkontaktbringen mit einem zweiphasigen Kühlmedium, das eine wässrige und eine organische Phase umfasst. Diese Stufe wird nachfolgend auch als Quench bezeichnet. Dieser Quench kann aus nur einer Stufe (3 in Figuren 1 bis 3) oder aus mehreren Stufen bestehen (beispielsweise 3, 8 in den Figuren 1 bis 3). Der Produktgasstrom 2 wird also direkt mit einem zweiphasigen Kühlmedium 6 in Kontakt gebracht und dadurch gekühlt. Die organische Phase enthält organische Lösungsmittel, bevorzugt aromatische Kohlenwasserstoffe, besonders bevorzugt Toluol, o-Xylol, m-Xylol, p-Xylol, Mesitylen, alle möglichen Konstitutionsisomere von Mono-, Di- und Triethylbenzol oder Gemische daraus, verwendet. Bevorzugt sind weiterhin aromatische Kohlenwasserstoffe mit einem Siedepunkt bei 1013, 25 hPa von über 120 °C oder Gemische daraus.

Im Allgemeinen hat das Produktgas 2, je nach Vorliegen und Temperaturniveau eines Wärmetauschers vor dem Quench 3, eine Temperatur von 100 bis 440 °C. Das Produktgas wird in der Quenchstufe 3 mit dem Kühlmedium aus wässriger und organischer Phase in Kontakt gebracht. Hierbei wird das Kühlmedium bevorzugt durch eine Düse eingebracht werden, um eine möglichst effiziente Durchmischung der wässrigen und der organischen Phase einerseits und des zweiphasigen Kühlmediums mit dem Produktgas andererseits zu erreichen. Zum gleichen Zweck können in der Quenchstufe Einbauten, wie zum Beispiel weitere Düsen, eingebracht werden, die das Produktgas und das Kühlmedium gemeinsam passieren. Der Kühlmitteleinlass in den Quench ist so ausgelegt, dass ein Verstopfen durch Ablagerungen im Bereich des Kühlmitteleinlasses minimiert wird.

Im Allgemeinen wird das Produktgas 2 in der ersten Quenchstufe 3 auf 5 bis 180 °C, vorzugsweise auf 30 bis 130 °C und noch mehr bevorzugt auf 50 bis 110 °C gekühlt. Die Temperatur des Kühlmediums 6 am Einlass kann im Allgemeinen 5 bis 200 °C, bevorzugt 20 bis 120°C, insbesondere bevorzugt 30 bis 90°C betragen. Der Druck in der ersten Quenchstufe 3 ist nicht besonders eingeschränkt, beträgt aber im Allgemeinen 0,01 bis 5 bar (ü), bevorzugt 0,1 bis 2 bar (ü) und besonders bevorzugt 0,2 bis 3 bar (ü). Wenn größere Mengen hochsiedende Nebenprodukte im Produktgas vorhanden sind, kann es leicht zur Polymerisation von hochsiedenden Nebenprodukten und zu Ablagerungen von Feststoffen, die durch hochsiedende Nebenprodukte in diesem Verfahrensabschnitt verursacht werden, kommen. Im Allgemeinen ist die Quenchstufe 3 als Kühlturm ausgestaltet. Das im Kühlturm eingesetzte Kühlmedium 6 wird zirkulierend in einem Quenchumlauf eingesetzt. Die Zirkulation kann durch eine geeignete Pumpe gewährleistet werden. Die Temperatur des Kühlmediums im Quenchumlauf kann gegebenenfalls durch einen Wärmetauscher kontrolliert werden. Der Kreislaufstrom des Kühlmediums in Liter pro Stunde, bezogen auf den Massenstrom an Butadien in Gramm pro Stunde, kann im Allgemeinen 0,0001 bis 51/g, bevorzugt 0,001 bis 11/g und besonders bevorzugt 0,002 bis 0,21/g betragen.

Die Temperatur des zweiphasigen Kühlmediums 6 im Sumpf kann im Allgemeinen 15 bis 210°C, bevorzugt 25 bis 130°C, insbesondere bevorzugt 35 bis 95°C betragen. Je nach Temperatur, Druck und Wassergehalt des Produktgases 2 kann es in der ersten Quenchstufe 3 zusätzlich zur Kondensation von Wasser kommen. Da die Beladung der organischen Phase und der wässrigen Phase mit Nebenkomponenten im Laufe der Zeit zunimmt, kann ein Teil des Kühlmediums aus dem Umlauf als Purgestrom 6b abgezogen und die Umlaufmenge durch Zugabe von schwächer beladener organischer Phase 5a und von schwächer beladener wässriger Phase 4a konstant gehalten werden. Das Verhältnis von Ablaufmenge und Zugabemenge hängt von der Dampfbeladung des Produktgases und der Produktgastemperatur am Ende der erste Quenchstufe ab. Die Orte der Zu- und Abläufe sind nicht weiter eingeschränkt. Sie können beispielsweise vor oder hinter der Pumpe oder dem Wärmetauscher befinden.

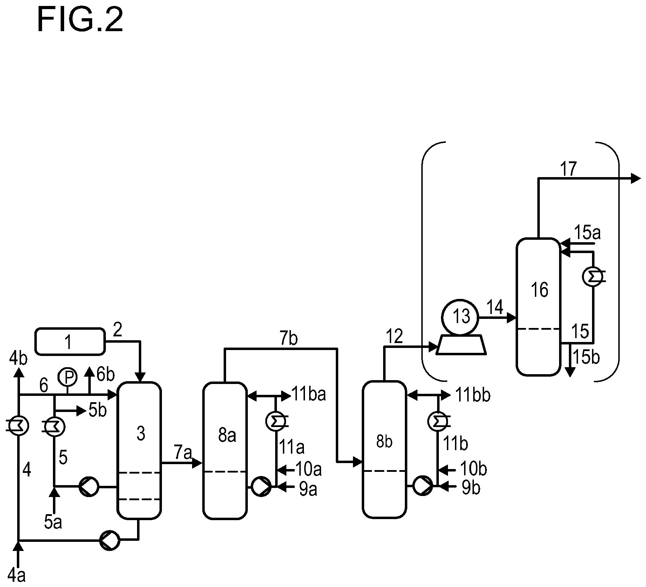

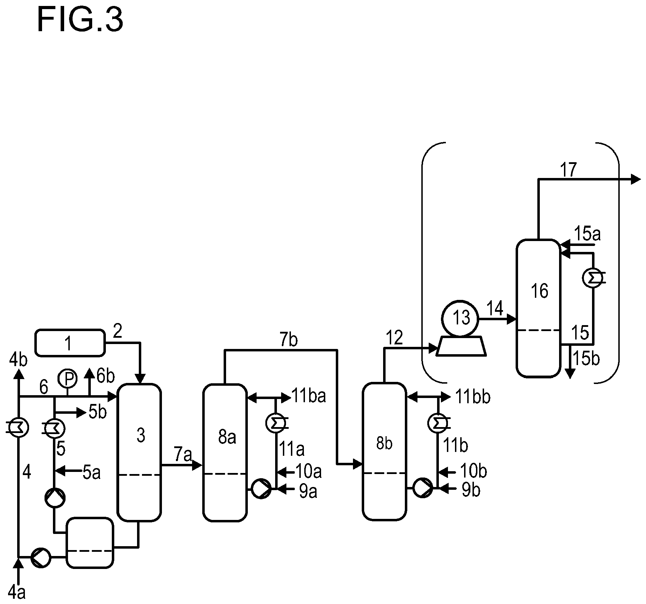

Im Sumpf der Quenchstufe 3 kann sich eine überwiegend wässrige Phase 4 ausbilden, welche zusätzlich wasserlösliche Nebenkomponenten enthalten kann. Diese kann, wie in Figur 2 gezeigt, aus dem Sumpf der Quenchstufe 3 abgezogen und zurückgeführt werden. Die wässrige Phase 4 kann auch, wie in Figur 3 gezeigt, in einem zusätzlichen Phasenscheider abgetrennt werden. Dieser kann sich beispielsweise innerhalb des Quenchumlaufs befinden. Die wässrige Phase 4 wird zumindest teilweise in den Quench zurückgeführt. Auch die organische Phase 5 wird zumindest teilweise in den Quench zurückgeführt. Anstelle oder zusätzlich zum Purgestrom 6b können auch ein Wasser-Purgestrom 4b und ein Organik-Purgestrom 5b abgetrennt werden.

In einer bevorzugten Ausführungsform ist der Quench zweistufig (umfassend die Stufen 3 und 8a gemäß Figuren 1 bis 3), d.h. die Stufe Ca) und umfasst zwei Abkühlstufen Ca1) und Ca2), in denen der Produktgasstrom b mit dem Abkühlmedium in Kontakt gebracht wird. Erfindungsgemäß ist zumindest das Kühlmedium in der ersten Quenchstufe zweiphasig. Die zwei Quenchstufen können sich in getrennten Kühltürmen oder in einem gemeinsamen Kühlturm befinden.

Dabei wird der abgekühlte und eventuell an Nebenkomponenten abgereicherte Produktgasstrom 7a einer zweiten Quenchstufe 8a zugeführt. In dieser wird er nun erneut mit einem Kühlmedium 11a in Kontakt gebracht. Das Kühlmedium 11a kann zweiphasig sein und eine wässrige Phase und eine organische Phase enthalten. Es kann aber auch überwiegend oder ausschließlich aus einem organischen Lösungsmittel bestehen.

Bevorzugt enthält das organische Lösungsmittel aromatische Kohlenwasserstoffe, besonders bevorzugt Toluol, o-Xylol, m-Xylol, p-Xylol, Mesitylen, alle möglichen Konstitutionsisomere von Mono-, Di- und Triethylbenzol sowie alle möglichen Konstitutionsisomere von Mono-, Di- und Triisopropylbenzol oder Gemische daraus. Bevorzugt sind weiterhin aromatische Kohlenwasserstoffe mit einem Siedepunkt bei 1013,25 hPa von über 120 °C oder Gemische daraus. Bevorzugt handelt es sich um das gleiche organische Lösungsmittel wie in der ersten Quenchstufe.

Im Allgemeinen wird das Produktgas bis zum Gasausgang der zweiten Quenchstufe 8a auf 5 bis 100°C, vorzugsweise auf 15 bis 85°C und bevorzugt auf 20 bis 70°C gekühlt. Das Kühlmittel kann im Gegenstrom zum Produktgas zugeführt werden. In diesem Fall kann die Temperatur des Kühlmittelmediums 11a am Kühlmitteleinlass 5 bis 100°C, bevorzugt 15 bis 85°C, insbesondere bevorzugt 30 bis 70°C betragen. Der Druck in der zweiten Quenchstufe 8a ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Die zweite Quenchstufe 8a ist bevorzugt als Kühlturm ausgestaltet. Das im Kühlturm eingesetzte Kühlmedium 11a wird zirkulierend in einem Quenchumlauf eingesetzt. Der Kreislaufstrom des Kühlmediums 11a in Liter pro Stunde, bezogen auf den Massenstrom an Butadien in Gramm pro Stunde, kann im Allgemeinen 0,0001 bis 5 1/g, bevorzugt 0,001 bis 11/g und besonders bevorzugt 0,002 bis 0,21/g betragen.

Da die Beladung des Kühlmediums 11a mit Nebenkomponenten im Laufe der Zeit zunimmt, kann ein Teil des Kühlmediums aus dem Umlauf als Purgestrom 11ba abgezogen und die Umlaufmenge durch Zugabe von weniger beladener organischer Phase 10a und gegebenenfalls von weniger beladener wässriger Phase 9a konstant gehalten werden.

Die Temperatur des Kühlmediums 11a im Sumpf kann im Allgemeinen 20 bis 210°C, bevorzugt 35 bis 120°C, insbesondere bevorzugt 45 bis 85°C betragen. Je nach Temperatur, Druck und Wassergehalt des Produktgases 7a kann es in der zweiten Quenchstufe 8a zusätzlich zur Kondensation von Wasser kommen. In diesem Falle kann sich im Sumpf eine zusätzliche wässrige Phase bilden. Die wässrige Phase kann auch in einem zusätzlichen Phasenscheider abgetrennt werden. Dieser kann sich beispielsweise innerhalb des Quenchumlaufs befinden. Die wässrige Phase kann abgezogen werden oder zumindest teilweise in den Quench zurückgeführt werden. Alternativ kann sich der Phasenscheider beispielsweise im Purgestrom 11ba befinden.

Die wässrige Phase kann zumindest teilweise als Purgestrom abgezogen oder zumindest teilweise in den Quench zurückgeführt werden. Ebenso kann die organische Phase zumindest teilweise als Purgestrom abgezogen oder zumindest teilweise in den Quench zurückgeführt werden.

Die Orte der Zu- und Abläufe in den Umläufen der jeweiligen Quenchstufen sind nicht weiter eingeschränkt. Sie können sich beispielsweise vor oder hinter der Pumpe oder dem Wärmetauscher befinden. Weiterhin ist der Ort der oder des Wärmetauschers im Quenchumlauf nicht weiter eingeschränkt. Im Falle von teilweise phasengetrennten Quenchumläufen können sich Wärmetauscher in einem oder beiden Umläufen befinden oder erst in den wieder vereinten Umläufen. Alternativ kann auf einen Wärmetauscher gänzlich verzichtet werden und die Quenchkühlung alleine durch Verdampfung des Kühlmittels geschehen. Weiterhin ist der Ort der Umlaufpumpen nicht weiter eingeschränkt. Im Falle eines Phasenscheiders im Umlaufstrom kann sich beispielsweise eine Pumpe vor dem Phasenscheider befinden oder jeweils eine Pumpe in jedem der phasengetrennten Umläufe.

Um einen möglichst guten Kontakt von Produktgas und Kühlmedium zu erreichen, können Einbauten in der zweiten Quenchstufe 8a vorhanden sein. Solche Einbauten umfassen zum Beispiel Glocken-, Zentrifugal- und/oder Siebböden, Kolonnen mit strukturierten Packungen, z.B. Blechpackungen mit einer spezifischen Oberfläche von 100 bis 1000 m$^2$/m$^3$ wie Mellapak® 250 Y, und Füllkörperkolonnen.

Die Kühlmittel-Umläufe der Quenchstufen können sowohl voneinander getrennt als auch miteinander verbunden sein. So kann beispielsweise ein Teil des Stroms 11ba dem Strom 6 zugeführt werden und die Ströme 4a und/oder 5a zumindest teilweise ersetzen. Die gewünschte Temperatur der Umlaufströme kann über geeignete Wärmetauscher eingestellt werden.

In einer bevorzugten Ausführungsform der Erfindung wird also die Abkühlstufe Ca) zweistufig durchgeführt, wobei das mit Nebenkomponenten beladene organische Lösungsmittel der zweiten Stufe Ca2) in die erste Stufe Ca1) geführt wird. Das der zweiten Stufe Ca2) entnommene organische Lösungsmittel enthält weniger Nebenkomponenten als das der ersten Stufe Ca1) entnommene organische Lösungsmittel.

Die Stufe Ca) kann auch mehrstufig in Stufen Ca1) bis Can), besonders bevorzugt dreistufig in drei Stufen Ca1), Ca2) und Ca3) durchgeführt werden. Dabei kann zumindest ein Teil des Kühlmediums nach Durchlaufen der dritten Stufe Ca3) als Abkühlmittel der zweiten Stufe Ca2) zugeführt werden.

In einer besonders bevorzugten Ausführungsform ist der Quench dreistufig ausgebildet (umfassend die Stufen 3, 8a und 8b gemäß Figuren 1 bis 3), d.h. die Stufe Ca) umfasst drei Abkühlstufen Ca1), Ca2) und Ca3), in denen der Produktgasstrom b mit dem Abkühlmedium in Kontakt gebracht wird. Erfindungsgemäß ist zumindest das Kühlmedium in der ersten Quenchstufe zweiphasig. Die drei Abkühlstufen können sich in getrennten Kühltürmen oder in einem gemeinsamen Kühlturm befinden.

Dabei wird der abgekühlte und eventuell an Nebenkomponenten abgereicherte Produktgasstrom 7a einer zweiten Quenchstufe 8a und der eventuell an Nebenkomponenten weiter abgereicherte Produktgasstrom 7b einer dritten Quenchstufe 8b zugeführt. In diesen Quenchstufen wird er nun erneut mit einem Kühlmedium 11b in Kontakt gebracht. Das Kühlmedium 11b kann zweiphasig sein und eine wässrige Phase und eine organische Phase enthalten. Es kann aber auch überwiegend oder ausschließlich aus einem organischen Lösungsmittel bestehen.

Bevorzugt handelt es sich in allen drei Quenchstufen um das gleiche organische Lösungsmittel.

Die Kühlmittel-Umläufe der drei Quenchstufen können sowohl voneinander getrennt als auch miteinander verbunden sein.

In einer besonders bevorzugten Ausführungsform der Erfindung wird also die Abkühlstufe Ca) dreistufig durchgeführt, wobei das mit Nebenkomponenten beladene organische Lösungsmittel der zweiten Stufe Ca2) in die erste Stufe Ca1) geführt wird und das mit Nebenkomponenten schwächer beladene organische Lösungsmittel der dritten Stufe Ca3) in die zweite Stufe Ca2) geführt wird.

In einer weiteren Ausführungsform wird in der dritten Abkühlstufe Ca3) ein frisches Kühlmedium aus einem organischen Lösungsmittel oder einer Mischung aus organischem Lösungsmittel und Wasser, wobei dieses Kühlmedium noch nicht mit den Nebenkomponenten beladen ist, in einfachem Durchlauf im Gegenstrom in die Abkühlstufe eingespeist. Da das frische Kühlmedium noch nicht mit den in den Quenchstufen zu entfernenden Nebenkomponenten beladen ist, wird eine weitere Reduzierung der im Produktgas nicht erwünschten Nebenkomponenten im Kopfprodukt des Kühlturms erreicht.

Um die für die Auslegung des Kühlturms erforderliche Flüssigkeitsbelastung in der Abkühlstufe Ca3) zu gewährleisten, kann der Durchmesser dieser Abkühlstufe Ca3) kleiner gewählt werden als der Durchmesser der Abkühlstufen Ca1) und Ca2). Falls die erforderliche Flüssigkeitsbelastung in der Abkühlstufe Ca3) durch Reduzierung des Durchmessers nicht erreicht werden kann, wird die Flüssigkeitsbelastung in diesem Abschnitt durch Umpumpen des Kühlmediums entsprechend erhöht.

In einer Ausführungsform der Erfindung ist die erste Abkühlstufe Ca1) parallel und doppelt umschaltbar ausgeführt. Im Normalbetrieb wird nur eine der beiden parallelen Abkühlstufen betrieben, während die andere für Reinigungsarbeiten außer Betrieb ist oder als Reserve zur Verfügung steht.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende und andere Substanzen, welche im Quench nicht vom Produktgas abgetrennt werden, durch weitere bauliche Maßnahmen, wie beispielsweise weitere Gaswäschen, aus dem Produktgas entfernt werden.

Es wird ein Gasstrom 12 erhalten, in der n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide, Inertgase und Teile des im Quench verwendeten Lösungsmittel enthält. Weiterhin können in diesem Gasstrom 12 Spuren von hochsiedenden Komponenten verbleiben, welche im Quench nicht quantitativ abgetrennt wurden.

Anschließend wird der Gasstrom b aus dem Abkühlschritt Ca), der an hochsiedenden Nebenkomponenten abgereichert ist, im Schritt Cb) in mindestens einer Kompressionsstufe Cba) und bevorzugt in mindestens einer Abkühlstufe Cbb) durch Inkontaktbringen mit einem organischen Lösungsmittel als Abkühlmittel abgekühlt.

Der Produktgasstrom 12 aus dem Kühlmittel-Quench (3 oder bevorzugt 3 und 8a oder bevorzugt 3, 8a und 8b) wird in mindestens einer Kompressionsstufe 13 komprimiert und nachfolgend in dem Kühlapparat 16 weiter abgekühlt.

Die Kompression und Kühlung des Gasstroms 12 kann ein- oder mehrstufig (n-stufig) erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60°C abgekühlt wird. Die Kühlung kann durch direkten oder indirekten Wärmeaustausch erfolgen.

Um den Strom 14 direkt zu kühlen und/oder um weitere Nebenkomponenten aus dem Strom 14 zu entfernen, wird der Strom 14 mit einem Kühlmittel 15 in Kontakt gebracht. Das Kühlmedium 15 kann einphasig oder zweiphasig ein und eine wässrige Phase und eine organische Phase enthalten. Die organische Phase enthält in einer bevorzugten Ausführung das gleiche organische Lösemittel wie die Quenchkühlmittel 6, 11a und 11b. Durch die Kühlung kondensiert Wasser sowie im Quench verwendetes organisches Lösemittel und gegebenenfalls weitere Nebenkomponenten aus. Da die Beladung des Kühlmittels 15 mit Nebenkomponenten im Laufe der Zeit zunimmt, kann ein Teil des beladenen Kühlmittels als Strom 15b aus dem Umlauf abgezogen werden und die Umlaufmenge des Kühlmittels durch Zugabe von weniger beladenem Kühlmittel 15a konstant gehalten werden.

Das Kühlmittel 15 kann in einem Wärmetauscher abgekühlt und als Kühlmittel in den Apparat 16 rückgeführt werden.

Der Kondensatstrom 15b kann in den Strom 5a und/oder 10a und/oder 10b zugeführt und damit in den Kreislaufstrom 6 und/oder 11a und/oder 11b des Quenches zurückgeführt werden. Dadurch können die im Kondensatstrom 15a absorbierten $C_4$-Komponenten wieder in den Gasstrom gebracht und damit die Ausbeute erhöht werden.

Es verbleibt ein Gasstrom 17 enthaltend Butadien, 1-Buten, 2-Butene, Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase. Weiterhin kann dieser Produktgasstrom noch Spuren von hochsiedenden Komponenten enthalten.

Geeignete Verdichter sind beispielsweise Turbo-, Drehkolben- und Hubkolbenverdichter. Die Verdichter können beispielsweise mit einem Elektromotor, einem Expander oder einer Gas oder Dampfturbine angetrieben werden. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt mit organischem Lösungsmittel gespülten Wärmetauschern oder organischen Quenchstufen, die beispielsweise als Rohrbündel-, Spiral oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

Der Butadien, n-Butene, Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase und gegebenenfalls Spuren von Nebenkomponenten enthaltende Gasstrom 17 wird als Ausgangsstrom der weiteren Aufbereitung zugeführt.

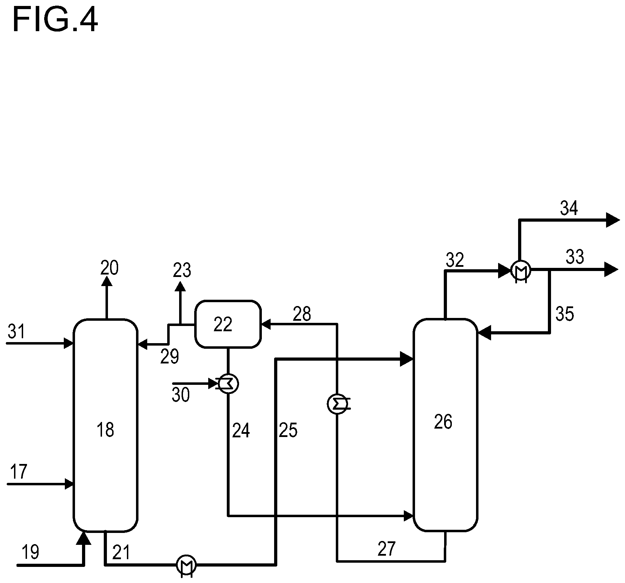

In einem, in Figur 4 dargestellten Schritt D) werden nicht kondensierbare und leicht siedende Gasbestandteile, umfassend Sauerstoff, leicht siedenden Kohlenwasserstoffe (Methan, Ethan, Ethen, Propan, Propen), Kohlenstoffoxide und Inertgase, in einer Absorptionskolonne als Gasstrom aus dem Prozessgasstrom 17 durch Absorption der $C_4$-Kohlenwasserstoffe in einem hochsiedenden Absorptionsmittel (29 und/oder 31) und nachfolgender Desorption der $C_4$-Kohlenwasserstoffe abgetrennt. Vorzugsweise umfasst der Schritt D), wie in Figur 4 dargestellt, die Schritte Da) bis Dc):

Da) Absorption der $C_4$-Kohlenwasserstoffe umfassend Butadien und n-Butene in einem hochsiedenden Absorptionsmittel (29 und/oder 31), wobei ein mit $C_4$-Kohlenwasserstoffen beladener Absorptionsmittelstrom und der Gasstrom 20 erhalten werden,

Db) Entfernung von Sauerstoff aus dem mit $C_4$-Kohlenwasserstoffen beladenen Absorptionsmittelstrom aus Schritt Da) durch Strippung mit einem nicht kondensierbaren Gasstrom 19 wobei ein mit $C_4$-Kohlenwasserstoffen beladener Absorptionsmittelstrom 21 erhalten wird und

Dc) Desorption der $C_4$-Kohlenwasserstoffe aus dem beladenen Absorptionsmittelstrom, wobei ein $C_4$-Produktgasstrom 32 erhalten wird, der im Wesentlichen aus $C_4$-Kohlenwasserstoffen besteht.

Dazu wird in der Absorptionsstufe 18 der Gasstrom 17 mit einem inerten Absorptionsmittel in Kontakt gebracht und werden die $C_4$-Kohlenwasserstoffe in dem inerten Absorptionsmittel absorbiert, wobei ein mit $C_4$-Kohlenwasserstoffen beladenes Absorptionsmittel und ein die übrigen Gasbestandteile enthaltendes Abgas 20 erhalten werden. In einer Desorptionsstufe werden die $C_4$-Kohlenwasserstoffe aus dem hochsiedenden Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z.B. Blechpackungen mit einer spezifischen Oberfläche von 100 bis 1000 m$^2$/m$^3$ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, n-Butene und die leichtsiedenden und nicht kondensierbaren Gasbestandteile enthaltende Gasstrom 17 zugeführt. Im oberen Bereich der Absorptionskolonne wird das hochsiedende Absorptionsmittel (29 und/oder 31) aufgegeben.

In der Absorptionsstufe eingesetzte inerte Absorptionsmittel sind im Allgemeinen hochsiedende unpolare Lösungsmittel, in denen das abzutrennende $C_4$-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische $C_8$- bis $C_{18}$-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen $C_1$-$C_8$-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

In einer bevorzugten Ausführungsform in der Absorptionsstufe Da) wird das gleiche Lösungsmittel wie in der Abkühlstufe Ca) eingesetzt.

Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. In der bevorzugten Ausführungsform wird als Lösungsmittel für die Absorption Toluol, o-Xylol, m-Xylol, p-Xylol, Mesitylen oder Gemische daraus eingesetzt.

Am Kopf der Absorptionskolonne 18 wird ein Abgasstrom 20 abgezogen, der im Wesentlichen Sauerstoff, leicht siedende Kohlenwasserstoffe (Methan, Ethan, Ethen, Propan, Propen), gegebenenfalls $C_4$-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom kann teilweise dem ODH-Reaktor zugeführt werden. Damit lässt sich zum Beispiel der Eintrittsstrom des ODH-Reaktors auf den gewünschten $C_4$-Kohlenwasserstoffgehalt einstellen.

Am Sumpf der Absorptionskolonne werden in einer weiteren Kolonne durch die Spülung mit einem Gas 19 Reste von im Absorptionsmittel gelöstem Sauerstoff ausgetragen. Der verbleibende Sauerstoffanteil soll so klein sein, dass der, die Desorptionskolonne verlassende und Butan, Buten sowie Butadien enthaltende Strom 32 nur noch maximal 100 ppm Sauerstoff enthält.

Das Ausstrippen des Sauerstoffs in Schritt Db) kann in jeder beliebigen, dem Fachmann bekannten geeigneten Kolonne durchgeführt werden. Das Strippen kann durch einfaches Durchleiten von nicht kondensierbaren Gasen, vorzugsweise Inertgasen wie Stickstoff, durch die beladene Absorptionslösung erfolgen. Mit ausgestrippte $C_4$-Kohlenwasserstoffe werden im oberen Teil der Absorptionskolonne 18 zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil erfindungsgemäß gleich ist, kann diese direkte Kopplung erfolgen. Geeignete Stippkolonnen sind z.B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 $m^2/m^3$ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der mit $C_4$-Kohlenwasserstoffen beladene Absorptionsmittelstrom 21 kann in einem Wärmetauscher erwärmt werden und als Strom 25 anschließend in eine Desorptionskolonne 26 geleitet werden. In einer Verfahrensvariante wird der Desorptionsschritt Dc) durch Entspannung und/oder Erhitzen des beladenen Absorptionsmittels durchgeführt. Bevorzugte Verfahrensvariante ist die Nutzung eines Dampfstroms 24, der im Sumpf der Desorptionskolonne 26 zugeführt wird.

Das in der Desorptionsstufe regenerierte Absorptionsmittel wird als Strom 27 zusammen mit dem kondensierten Wasser aus der Desorptionskolonne 26 entnommen. Dieses zweiphasige Gemisch kann in einem Wärmetauscher abgekühlt werden und als Strom 28 in einem Dekanter 22 in einen wässrigen und einen Absorbensstrom 29 getrennt werden. Der Absorbensstrom 29 wird wieder der Absorberkolonne 18 zugeführt werden, während der wässrige Strom in einem Verdampfer verdampft wird und damit der Strom 24 erzeugt wird. Zusätzlich oder alternativ kann noch zusätzliches Wasser (Strom 30) im Verdampfer verdampft werden.

Im Prozessgasstrom befindliche Leichtsieder wie beispielsweise Ethan oder Propan sowie schwersiedende Komponenten wie Benzaldehyd, Maleinsäureanhydrid und Phthalsäureanhydrid, können sich im Kreislaufstrom anreichern. Um die Anreicherung zu begrenzen, kann ein Purge-strom 23 abgezogen werden.

Der im Wesentlichen aus n-Butan, n-Butenen und Butadien bestehende $C_4$-Produktgasstrom 32 enthält im Allgemeinen 20 bis 80 Vol.-% Butadien, 0 bis 80 Vol.-% n-Butan, 0 bis 10 Vol.-% 1-Buten, und 0 bis 50 Vol.-% 2-Butene, wobei die Gesamtmenge 100 Vol.-% ergibt. Weiterhin können geringe Mengen an Iso-Butan enthalten sein.

Ein Teil des kondensierten, hauptsächlich $C_4$-Kohlenwasserstoffe enthaltenen Kopfaustrags der Desorptionskolonne wird als Strom 35 in den Kolonnenkopf zurückgeführt, um die Trennleistung der Kolonne zu erhöhen.

Die, den Kondensator verlassenden, flüssigen (Strom 33) bzw. gasförmigen (Strom 34) $C_4$-Produktströme werden anschließend durch Extraktivdestillation im Schritt E) mit einem für Butadien selektiven Lösungsmittel in einen Butadien und das selektive Lösungsmittel enthaltenden Stoffstrom und einen n-Butene enthaltenden Stoffstrom aufgetrennt.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der $C_4$-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu $C_4$-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250°C, insbesondere bei einer Temperatur im Bereich von 110 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder isoButyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom der Extraktivdestillationskolonne enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom bis 100 Vol-% n-Butan, 0 bis 50 Vol-% 2-Buten und 0 bis 3 Vol-% weitere Bestandteile wie Isobutan, Isobuten, Propan, Propen und $C_5$ -Kohlenwasserstoffe.

Der im Wesentlichen aus n-Butan und 2-Buten bestehende Strom kann ganz oder teilweise dem $C_4$-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden.

In einem Schritt F) wird der Butadien und das selektive Lösungsmittel enthaltende Stoffstrom in einen im Wesentlichen aus dem selektiven Lösungsmittel bestehenden Stoffstrom und einen Butadien enthaltenden Stoffstrom destillativ aufgetrennt.

Der am Sumpf der Extraktivdestillationskolonne gewonnene Stoffstrom enthält im Allgemeinen das Extraktionsmittel, Wasser, Butadien und in geringen Anteilen Butene und Butan und wird einer Destillationskolonne zugeführt. In dieser kann über Kopf oder als Seitenabzug Butadien gewonnen werden. Am Sumpf der Destillationskolonne fällt ein Extraktionsmittel und gegebenenfalls Wasser enthaltender Stoffstrom an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

Falls das Butadien über einen Seitenabzug gewonnen wird, wird die so abgezogene Extraktionslösung in eine Desorptionszone überführt, wobei aus der Extraktionslösung das Butadien nochmals desorbiert und rückgewaschen wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Als Einbauten sind Packungen, Böden oder Füllkörper vorgesehen. Die Destillation wird vorzugsweise bei einer Sumpftemperatur im Bereich von 100 bis 300°C, insbesondere im Bereich von 150 bis 200°C und einer Kopftemperatur im Bereich von 0 bis 70°C, insbesondere im Bereich von 10 bis 50°C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

**Beispiele**

Dehydrierversuche

In einem Miniplant-Reaktor wurden Dehydrierungsversuche durchgeführt. Der Miniplant-Reaktor war ein Salzbadreaktor mit einer Länge von 500 cm und einem Innendurchmesser von 29,7 mm und einer Thermohülse mit einem Außendurchmesser von 6 mm. Auf einem Katalysatorstuhl saß eine 10 cm lange Nachschüttung bestehend aus 60 g Steatitringen der Geometrie 7 mm x 7 mm x 4 mm (Außendurchmesser x Länge x Innendurchmesser). Auf diese folgten 2705 g eines unverdünnten Schalenkatalysators (Aktivmassegehalt 19,6 Gew.-%; Schütthöhe 384 cm, 2552 ml Schüttvolumen im Reaktor) in Form von Hohlzylindern der Abmessungen 7 mm x 3 mm x 4 mm (Außendurchmesser x Länge x Innendurchmesser). An die Katalysatorschüttung schloss sich eine 85 cm lange Vorschüttung bestehend aus 494 g Steatitringen der Geometrie 7 mm x 7 mm x 4 mm (Außendurchmesser x Länge x Innendurchmesser) an.

Das Reaktionsrohr wurde auf seiner gesamten Länge mit einem es umfließenden Salzbad temperiert. Als Reaktionsgasgemisch wurde ein Gemisch umfassend insgesamt 8 Vol.-% 1-, cis-2- und trans-2-Butenen, geringe Mengen i-Buten, 2 Vol.-% Butane (n- und iso-Butan), 12 Vol.-% Sauerstoff, 5 Vol.-% Wasser und Rest Stickstoff eingesetzt. Die Belastung des Reaktionsrohres betrug 5500 Nl/h Gesamtgas. Die Temperatur des Salzes betrug nach dem Anfahren während des stabilen Betriebs 374 °C. Der Butene-Umsatz lag bei 85 % und die Selektivität zu Butadien ebenfalls bei etwa 85 %. Detektierte Nebenkomponenten umfassen Essigsäure, Methacrolein, Methylvinylketon, Methylethylketon, Crotonaldehyd, Acrylsäure, Propionsäure, Methacrylsäure, Vinylcyclohexan, Maleinsäureanhydrid, Ethylbenzol, Styrol, Furanon, Benzaldehyd, Benzoesäure, Phthalsäureanhydrid, Fluorenon, Anthrachinon, Formaldehyd, Kohlenmonoxid und Kohlendioxid.

Das ODH Abgas wurde mittels eines Wärmetauschers auf 180 °C abgekühlt. Es kann direkt auf eine Fackel gefahren werden oder seitlich in den Kopf einer Quench-Kolonne eingeführt (siehe Schema). Die Quenchkolonne ist 920 mm lang und hat einen Innendurchmesser von 56 mm. Nach 300 und 610 mm ist jeweils eine Venturidüse mit einem zentralen Lochdurchmesser von 8 mm eingebaut. Das Kühlmittel wird von oben durch eine Vollkegeldüse (Beispiel 1, Vergleichsbeispiel 1 und 2) oder 2 Vollkegeldüsen (Beispiele 2 und 3) (kleinster freier Querschnitt 1,15 mm) dem Quenchkopf zugeführt. Das Gas/Kühlmittel-Gemisch durchquert die Quenchkolonne durch die zwei Venturidüsen, wird im Sumpf der Quenchkolonne gesammelt und als Quenchumlauf mit Hilfe einer Pumpe durch einen Wärmetauscher zurück zum Kolonnenkopf gefördert. Der Druck auf der Druckseite der Pumpe wird gemessen. Ein Teil des beladenen Kühlmittels kann dem Umlauf entnommen werden. Weiterhin kann dem Umlauf Kühlmittel zugeführt werden.

Das Produktgas wird der weiteren Aufarbeitung zugeführt

Beispiel 1

Dem Umlaufstrom an Kühlmittel um die Quenchkolonne werden 910 g/h entionisiertes Wasser und 2017 g/h Mesitylen (1,3,5-Trimethylbenzol) zugeführt (siehe Tabelle). Der Umlaufstrom um die Quenchkolonne beträgt 60 l/h. Der Stand an Kühlmittel im Sumpf wird konstant gehalten, indem Kühlmittel aus dem Umlauf ausgeschleust wird.

Wenn der Reaktor einen stabilen Zustand erreicht hat, wird das Reaktorabgas von der Fackel in die Quenchkolonne umgeleitet. Nach wenigen Stunden hat das Kühlmittel kurz vor der Kegeldüse eine Temperatur von 75 °C, im Sumpf eine Temperatur von 71 °C. Die Temperatur des Gasraums über dem Kühlmittelsumpf wird mit 73 °C gemessen. Der Druck im Kühlmittelumlauf beträgt 1,5 bar Überdruck. Der Gehalt an Wasserdampf und Mesitylen im Produktgas, welches die Quenchkolonne verlässt wird per online GC gemessen. Das Phasenverhältnis im Quenchumlauf, ausgedrückt als Masse an Wasser zu Masse an Mesitylen, beträgt 0,45. Reaktor und Quench liefen über 150 Stunden bei nahezu konstantem Kühlmittelumlaufstrom, ohne dass es zu signifikanten Änderungen im Druck gekommen ist.

Vergleichsbeispiel 1

Dem Umlauf werden 2000 g/h Mesitylen (1,3,5-Trimethylbenzol) zugeführt. Der Umlaufstrom an Kühlmittel um die Quenchkolonne beträgt 60 l/h (siehe Tabelle). Die Menge an Kühlmittel im Sumpf der Kolonne wird konstant gehalten, indem Kühlmittel aus dem Umlauf ausgeschleust wird.

Wenn der Reaktor einen stabilen Zustand erreicht hat, wird das Reaktorabgas von der Fackel in die Quenchkolonne umgeleitet. Nach wenigen Stunden hat das Kühlmittel kurz vor der Kegeldüse eine Temperatur von 75 °C und im Sumpf der Quenchkolonne eine Temperatur von 71 °C. Die Temperatur des Gasraums über dem Kühlmittelsumpf wird mit 73 °C gemessen. Der Druck im Kühlmittelumlauf beträgt 1,5 bar Überdruck. Der Gehalt an Wasserdampf und Mesitylen im Produktgas, welches die Quenchkolonne verlässt, wird per online-GC gemessen. Das Phasenverhältnis im Quenchumlauf, ausgedrückt als Masse an Wasser zu Masse an Mesitylen, ist kleiner als 0.01.

Innerhalb kurzer Zeit beginnt der Druck im Umlauf zu steigen und der Umlaufstrom gleichzeitig zu fallen. Nach 17 h ist der Umlauffluss auf unter 40 l/h gefallen, was eine Notabschaltung der Anlage nach sich zieht. Beim Öffnen der Anlage werden feste Ablagerungen in der Kegeldüse festgestellt.

Vergleichsbeispiel 2

Dem Umlauf werden 1500 g/h entionisiertes Wasser zugeführt. Der Umlaufstrom an Kühlmittel um die Quenchkolonne beträgt 60 l/h (siehe Tabelle). Der Stand an Kühlmittel im Sumpf wird konstant gehalten, in dem Kühlmittel aus dem Umlauf ausgeschleust wird.

Wenn der Reaktor einen stabilen Zustand erreicht hat, wird das Reaktorabgas von der Fackel in die Quenchkolonne umgeleitet. Nach wenigen Stunden hat das Kühlmittel kurz vor der Kegeldüse eine Temperatur von 75 °C und im Kolonnensumpf eine Temperatur von 71 °C. Die Temperatur des Gasraums über dem Kühlmittelsumpf wird mit 73 °C gemessen. Der Druck im Kühlmittelumlauf beträgt 1,5 bar Überdruck. Der Gehalt an Wasserdampf und Mesitylen im Produktgas, welches die Quenchkolonne verlässt, wird per online-GC gemessen. Das Phasenverhältnis im Quenchumlauf, ausgedrückt als Masse an Wasser zu Masse an Mesitylen, ist größer als 185.

Innerhalb kurzer Zeit beginnt der Druck im Umlauf zu steigen und der Umlaufstrom gleichzeitig zu fallen. Nach 14 h ist der Umlauffluss auf unter 40 l/h gefallen, was eine Notabschaltung der Anlage nach sich zieht. Beim Öffnen der Anlage werden feste Ablagerungen in der Kegeldüse festgestellt.

Beispiel 2

Dem Umlaufstrom des Kühlmittels um die Quenchkolonne werden 1112 g/h entionisiertes Wasser und 2173 g/h Mesitylen (1,3,5-Trimethylbenzol) zugeführt (siehe Tabelle). Der Umlaufstrom um die Quenchkolonne beträgt jeweils 60 l/h durch 2 Vollkegeldüsen. Der Stand des Kühlmittels im Sumpf wird konstant gehalten, indem Kühlmittel aus dem Umlauf ausgeschleust wird.

Wenn der Reaktor einen stabilen Zustand erreicht hat, wird das Reaktorabgas von der Fackel in die Quenchkolonne umgeleitet. Nach wenigen Stunden hat das Kühlmittel kurz vor der Kegeldüse eine Temperatur von 75 °C, im Sumpf eine Temperatur von 73 °C. Die Temperatur des Gasraums über dem Kühlmittelsumpf wird mit 75 °C gemessen. Der Druck im Kühlmittelumlauf beträgt 1,5 bar Überdruck. Der Gehalt an Wasserdampf und Mesitylen im Produktgas, welches die Quenchkolonne verlässt, wird per online-GC gemessen. Das Phasenverhältnis im Quenchumlauf, ausgedrückt als Masse an Wasser zu Masse an Mesitylen, beträgt 0,53 : 1. Reaktor und Quench laufen über 150 Stunden bei nahezu konstantem Kühlmittelumlaufstrom, ohne dass es zu signifikanten Druckänderungen kommt.

Beispiel 3

Dem Umlaufstrom des Kühlmittels um die Quenchkolonne werden 1314 g/h entionisiertes Wasser und 2083 g/h Mesitylen (1,3,5-Trimethylbenzol) zugeführt (siehe Tabelle). Der Umlaufstrom um die Quenchkolonne jeweils 60 l/h durch 2 Vollkegeldüsen. Der Stand des Kühlmittels im Sumpf wird konstant gehalten, indem Kühlmittel aus dem Umlauf ausgeschleust wird.

Wenn der Reaktor einen stabilen Zustand erreicht hat, wird das Reaktorabgas von der Fackel in die Quenchkolonne umgeleitet. Nach wenigen Stunden hat das Kühlmittel kurz vor der Kegeldüse eine Temperatur von 75 °C, im Sumpf eine Temperatur von 73 °C. Die Temperatur des Gasraums über dem Kühlmittelsumpf wird mit 75 °C gemessen. Der Druck im Kühlmittelumlauf beträgt 1,5 bar Überdruck. Der Gehalt an Wasserdampf und Mesitylen im Produktgas, welches die Quenchkolonne verlässt, wird per online-GC gemessen. Das Phasenverhältnis im Quenchumlauf, ausgedrückt als Masse an Wasser zu Masse an Mesitylen, beträgt 0,99 : 1. Reaktor und Quench laufen über 150 Stunden bei nahezu konstantem Kühlmittelumlaufstrom, ohne dass es zu signifikanten Druckänderungen kommt.

Tabelle

| | Beispiel 1 | Vergleichsbeispiel 1 | Vergleichsbeispiel 2 | Beispiel 2 | Beispiel 3 |
|---|---|---|---|---|---|
| Zulauf Wasserdampf aus Reaktor [g/h] | 664 | 664 | 664 | 587 | 629 |
| Zulauf Wasser zum Umlauf [g/h] | 910 | - | 1500 | 1112 | 1314 |
| Ablauf Wasserdampf Quenchkolonne [g/h] | -1236 | >658 | -1236 | -1397 | -1418 |

(fortgesetzt)

| | Beispiel 1 | Vergleichsbeispiel 1 | Vergleichsbeispiel 2 | Beispiel 2 | Beispiel 3 |
|---|---|---|---|---|---|
| Ablauf Wasser aus Umlauf [g/h] | -338 | <6 | -928 | -302 | -525 |
| Zulauf Mesitylen zum Umlauf [g/h] | 2017 | 2000 | <5 | 2173 | 2083 |
| AblaufMesitlyendampf Quenchkolonne [g/h] | -1264 | -1264 | <5 | -1599 | -1553 |
| Ablauf Mesitylen aus Umlauf [g/h] | -753 | -736 | <5 | -574 | -530 |
| Phasenverhältnis im Umlauf [$g_{Was-ser}/g_{Mesitylen}$] | 0,45 | <0,01 | >185 | 0,53 | 0,99 |
| Umlaufstrom [l/h] | 60 | 60 | 60 | 2 x 60 | 2 x 60 |

**Patentansprüche**

1. Verfahren zur Herstellung von Butadien aus n-Butenen mit den Schritten:

    A) Bereitstellung eines n-Butene enthaltenden Einsatzgasstroms a;

    B) Einspeisung des n-Butene enthaltenden Einsatzgasstromes a und eines mindestens sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird;

    Ca) Abkühlung des Produktgasstroms b durch Inkontaktbringen mit einem Kühlmedium in mindestens einer Abkühlzone, wobei das Kühlmedium zumindest teilweise zurückgeführt wird und eine wässrige und eine organische Phase aus einem organischen Lösungsmittel aufweist, wobei das organische Lösungsmittel ausgewählt ist aus der Gruppe bestehend aus Toluol, o-, m- und p-Xylol, Mesitylen, Mono-, Di- und Triethylbenzol, Mono-, Di- und Triisopropylbenzol und Gemischen daraus und das Massenverhältnis der wässrigen Phase zur organischen Phase in dem Kühlmedium beim Einspeisen in die Abkühlzonen vor dem Inkontaktbringen mit dem Produktgasstrom von 0,15 : 1 bis 10 : 1 beträgt;

    Cb) Kompression des abgekühlten und gegebenenfalls an hochsiedenden Nebenkomponenten abgereicherten Produktgasstroms b in mindestens einer Kompressionsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;

    D) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der $C_4$-Kohlenwasserstoffe umfassend Butadien und n-Butene in einem Absorptionsmittel, wobei ein mit $C_4$-Kohlenwasserstoffen beladener Absorptionsmittelstrom und der Gasstrom d2 erhalten werden, und anschließende Desorption der $C_4$-Kohlenwasserstoffe aus dem beladenen Absorptionsmittelstrom, wobei ein $C_4$-Produktgasstrom d1 erhalten wird,

    E) Auftrennung des $C_4$-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;

    F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

2. Verfahren nach Anspruch 1, **dadurch gekennzeichnet, dass** das Massenverhältnis der wässrigen Phase zur organischen Phase in dem in Schritt (a) verwendeten Kühlmedium von 0,18 : 1 bis 2 : 1 beträgt..

3. Verfahren nach Anspruch 2, **dadurch gekennzeichnet, dass** die Stufe Ca) in drei Stufen Ca1), Ca2) und Ca3) in drei Abkühlzonen durchgeführt wird.

**4.** Verfahren nach Anspruch 3, **dadurch gekennzeichnet**, das ein mit hochsiedenden Nebenkomponenten beladenes organisches Lösungsmittel aus zweiten Stufe Ca2) in die erste Stufe Ca1) geführt wird und ein mit hochsiedenden Nebenkomponenten schwächer beladenes organisches Lösungsmittel aus der dritten Stufe Ca3) in die zweite Stufe Ca2) geführt wird.

**5.** Verfahren nach Anspruch 3 oder 4, **dadurch gekennzeichnet, dass** in die dritte Abkühlstufe Ca3) ein frisches Kühlmedium, das noch nicht mit den hochsiedenden Nebenkomponenten beladen ist, in einfachem Durchlauf im Gegenstrom in die Abkühlstufe eingespeist wird.

**6.** Verfahren nach einem der Ansprüche 3 bis 5, **dadurch gekennzeichnet, dass** der Durchmesser der Abkühlstufe Ca3) kleiner ist als der Durchmesser der Abkühlstufen Ca1) und Ca2).

**7.** Verfahren nach einem der Ansprüche 3 bis 6, **dadurch gekennzeichnet, dass** die erste Abkühlstufe Ca1) parallel und doppelt umschaltbar ausgeführt ist.

**8.** Verfahren nach einem der Ansprüche 1 bis 7, **dadurch gekennzeichnet, dass** das Kühlmedium durch eine oder mehrere Düsen in die Abkühlzonen eingespeist wird.

**9.** Verfahren nach Anspruch 8, **dadurch gekennzeichnet, dass** in der oder den Düsen eine Strömung erzeugt wird, in der die Reynoldszahl Re beider Phasen des Kühlmediums mindestens 100 beträgt.

**10.** Verfahren nach einem der Ansprüche 1 bis 5, **dadurch gekennzeichnet, dass** der volumenspezifische Leistungseintrag in das Kühlmedium mindestens $10^3$ W/m$^3$ beträgt.

**11.** Verfahren nach einem der Ansprüche 1 bis 10, **dadurch gekennzeichnet, dass** der Variationskoeffizient jeder Komponente des Kühlmediums beim Eintritt in die Abkühlzonen kleiner 1 beträgt.

**12.** Verfahren nach einem der Ansprüche 1 bis 11, **dadurch gekennzeichnet, dass** die Stufe Cb) mindestens eine Kompressionsstufe Cba) und mindestens eine Abkühlstufe Cbb) umfasst.

**13.** Verfahren nach Anspruch 12, **dadurch gekennzeichnet, dass** das Abkühlmittel der Abkühlstufe Cbb) das gleiche organische Lösungsmittel, das in der Stufe Ca) als organische Phase des Kühlmediums verwendet wird, enthält.

**14.** Verfahren nach einem der Ansprüche 1 bis 13, **dadurch gekennzeichnet, dass** die Stufe Cb) mehrere Kompressionsstufen Cba1) bis Cban) und Abkühlstufen Cbb1) bis Cbbn) umfasst.

**15.** Verfahren nach einem der Ansprüche 1 bis 14, **dadurch gekennzeichnet**, das Schritt D) die Schritte Da) bis Dc) umfasst:

Da) Absorption der $C_4$-Kohlenwasserstoffe umfassend Butadien und n-Butene in einem hochsiedenden Absorptionsmittel, wobei ein mit $C_4$-Kohlenwasserstoffen beladener Absorptionsmittelstrom und der Gasstrom d2 erhalten werden,
Db) Entfernung von Sauerstoff aus dem mit $C_4$-Kohlenwasserstoffen beladenen Absorptionsmittelstrom aus Schritt Da) durch Strippung mit einem nicht kondensierbaren Gasstrom, und
Dc) Desorption der $C_4$-Kohlenwasserstoffe aus dem beladenen Absorptionsmittelstrom, wobei ein $C_4$-Produktgasstrom d1 erhalten wird, der weniger als 100 ppm Sauerstoff umfasst.

**16.** Verfahren nach Anspruch 15, **dadurch gekennzeichnet, dass** das in Schritt Da) eingesetzte hochsiedende Absorptionsmittel ein aromatisches Kohlenwasserstofflösungsmittel ist.

**Claims**

**1.** A process for preparing butadiene from n-butenes, comprising the steps of:

A) providing an input gas stream a comprising n-butenes,
B) feeding the input gas stream a comprising n-butenes and a gas containing at least oxygen into at least one oxidative dehydrogenation zone and oxidatively dehydrogenating n-butenes to butadiene, giving a product gas stream b comprising butadiene, unconverted n-butenes, water vapor, oxygen, low-boiling hydrocarbons and high-boiling secondary components, with or without carbon oxides and with or without inert gases;

Ca) cooling the product gas stream b by contacting with a cooling medium in at least one cooling zone, the cooling medium being at least partly recycled and having an aqueous phase and an organic phase from an organic solvent, the organic solvent being selected from the group consisting of toluene, o-, m- and p-xylene, mesitylene, mono-, di- and triethylbenzene, mono-, di- and triisopropylbenzene and mixtures thereof, and the mass ratio of the aqueous phase to the organic phase in the cooling medium when it is fed into the cooling zones prior to the contacting with the product gas stream being from 0.15:1 to 10:1;

Cb) compressing the cooled product gas stream b which may have been depleted of high-boiling secondary components in at least one compression stage, giving at least one aqueous condensate stream c1 and one gas stream c2 comprising butadiene, n-butenes, water vapor, oxygen and low-boiling hydrocarbons, with or without carbon oxides and with or without inert gases;

D) removing uncondensable and low-boiling gas constituents comprising oxygen and low-boiling hydrocarbons, with or without carbon oxides and with or without inert gases, as gas stream d2 from the gas stream c2 by absorbing the $C_4$ hydrocarbons comprising butadiene and n-butenes in an absorbent, giving an absorbent stream laden with $C_4$ hydrocarbons and the gas stream d2, and then desorbing the $C_4$ hydrocarbons from the laden absorbent stream, giving a $C_4$ product gas stream d1,

E) separating the $C_4$ product stream d1 by extractive distillation with a butadiene-selective solvent into a stream e1 comprising butadiene and the selective solvent and a stream e2 comprising n-butenes;

F) distilling the stream e1 comprising butadiene and the selective solvent into a stream f1 consisting essentially of the selective solvent and a stream f2 comprising butadiene.

2. The process according to claim 1, wherein the mass ratio of the aqueous phase to the organic phase in the cooling medium used in step (a) is from 0.18:1 to 2:1.

3. The process according to claim 2, wherein stage Ca) is conducted in three stages Ca1), Ca2) and Ca3) in three cooling zones.

4. The process according to claim 3, wherein an organic solvent laden with high-boiling secondary components from the second stage Ca2) is conducted into the first stage Ca1), and an organic solvent less heavily laden with high-boiling secondary components from the third stage Ca3) is conducted into the second stage Ca2).

5. The process according to claim 3 or 4, wherein a fresh cooling medium as yet unladen with the high-boiling secondary components is fed into the third cooling stage Ca3) in single pass and in countercurrent into the cooling stage.

6. The process according to any of claims 3 to 5, wherein the diameter of the cooling stage Ca3) is less than the diameter of the cooling stages Ca1) and Ca2).

7. The process according to any of claims 3 to 6, wherein the first cooling stage Ca1) has a parallel and interchangeable configuration.

8. The process according to any of claims 1 to 7, wherein the cooling medium is fed into the cooling zones through one or more nozzles.

9. The process according to claim 8, wherein a flow is generated in the nozzle(s), in which the Reynolds number Re of the two phases of the cooling medium is at least 100.

10. The process according to any of claims 1 to 5, wherein the volume-specific power input into the cooling medium is at least $10^3$ $W/m^3$.

11. The process according to any of claims 1 to 10, wherein the coefficient of variation for each component of the cooling medium on entry into the cooling zones is less than 1.

12. The process according to any of claims 1 to 11, wherein stage Cb) comprises at least one compression stage Cba) and at least one cooling stage Cbb).

13. The process according to claim 12, wherein the coolant in the cooling stage Cbb) comprises the same organic solvent which is used in stage Ca) as the organic phase of the cooling medium.

**14.** The process according to any of claims 1 to 13, wherein stage Cb) comprises a plurality of compression stages Cba1) to Cban) and cooling stages Cbb1) to Cbbn).

**15.** The process according to any of claims 1 to 14, wherein step D) comprises steps Da) to Dc):

Da) absorbing the $C_4$ hydrocarbons comprising butadiene and n-butenes in a high-boiling absorbent, giving an absorbent stream laden with $C_4$ hydrocarbons and the gas stream d2,
Db) removing oxygen from the absorbent stream laden with $C_4$ hydrocarbons from step Da) by stripping with an uncondensable gas stream, and
Dc) desorbing the $C_4$ hydrocarbons from the laden absorbent stream, giving a $C_4$ product gas stream d1 comprising less than 100 ppm of oxygen.

**16.** The process according to claim 15, wherein the high-boiling absorbent used in step Da) is an aromatic hydrocarbon solvent.

**Revendications**

**1.** Procédé de fabrication de butadiène à partir de n-butènes comprenant les étapes suivantes :

A) la préparation d'un courant gazeux d'entrée contenant des n-butènes a ;
B) l'introduction du courant gazeux d'entrée contenant des n-butènes a et d'un gaz contenant au moins de l'oxygène dans au moins une zone de déshydrogénation oxydative, et la déshydrogénation oxydative des n-butènes en butadiène, un courant gazeux de produits b contenant du butadiène, des n-butènes non réagis, de la vapeur d'eau, de l'oxygène, des hydrocarbures de faible point d'ébullition, des composants secondaires de point d'ébullition élevé, éventuellement des oxydes de carbone et éventuellement des gaz inertes étant obtenu ;
Ca) le refroidissement du courant gazeux de produits b par mise en contact avec un milieu réfrigérant dans au moins une zone de refroidissement, le milieu réfrigérant étant au moins partiellement recyclé et comprenant une phase aqueuse et une phase organique constituée par un solvant organique, le solvant organique étant choisi dans le groupe constitué par le toluène, l'o-, le m- et le p-xylène, le mésitylène, le mono-, le di- et le triéthylbenzène, le mono-, le di- et le triisopropylbenzène et leurs mélanges, et le rapport en masse entre la phase aqueuse et la phase organique dans le milieu réfrigérant lors de l'introduction dans les zones de refroidissement avant la mise en contact avec le courant gazeux de produits étant de 0,15:1 à 10:1 ;
Cb) la compression du courant gazeux de produits b refroidi et éventuellement appauvri en composants secondaires de point d'ébullition élevé en au moins une étape de compression, au moins un courant de condensat aqueux c1 et un courant gazeux c2 contenant du butadiène, des n-butènes, de la vapeur d'eau, de l'oxygène, des hydrocarbures de faible point d'ébullition, éventuellement des oxydes de carbone et éventuellement des gaz inertes étant obtenus ;
D) la séparation des constituants gazeux non condensables et de faible point d'ébullition comprenant de l'oxygène, des hydrocarbures de faible point d'ébullition, éventuellement des oxydes de carbone et éventuellement des gaz inertes en tant que courant gazeux d2 à partir du courant gazeux c2 par absorption des hydrocarbures en $C_4$ comprenant le butadiène et les n-butènes dans un agent d'absorption, un courant d'agent d'absorption chargé avec des hydrocarbures en $C_4$ et le courant gazeux d2 étant obtenus, puis la désorption des hydrocarbures en $C_4$ du courant d'agent d'absorption chargé, un courant gazeux de produits en $C_4$ d1 étant obtenu,
E) la séparation du courant de produits en $C_4$ d1 par distillation extractive avec un solvant sélectif pour le butadiène en un courant de matières e1 contenant le butadiène et le solvant sélectif, et un courant de matières e2 contenant les n-butènes ;
F) la distillation du courant de matières e1 contenant le butadiène et le solvant sélectif en un courant de matières f1 essentiellement constitué par le solvant sélectif, et un courant de matières f2 contenant le butadiène.

**2.** Procédé selon la revendication 1, **caractérisé en ce que** le rapport en masse entre la phase aqueuse et la phase organique dans le milieu réfrigérant utilisé à l'étape (a) est de 0,18:1 à 2:1.

**3.** Procédé selon la revendication 2, **caractérisé en ce que** l'étape Ca) est réalisée en trois étapes Ca1), Ca2) et Ca3) dans trois zones de refroidissement.

**4.** Procédé selon la revendication 3, **caractérisé en ce qu'**un solvant organique chargé avec des composants secondaires de point d'ébullition élevé issu de la deuxième étape Ca2) est introduit dans la première étape Ca1), et un solvant organique moins chargé avec des composants secondaires de point d'ébullition élevé issu de la troisième étape Ca3) est introduit dans la deuxième étape Ca2).

5. Procédé selon la revendication 3 ou 4, **caractérisé en ce qu'**un milieu réfrigérant frais, qui n'est pas encore chargé avec les composants secondaires de point d'ébullition élevé, est introduit dans la troisième étape de refroidissement Ca3) en passage simple à contre-courant dans l'étape de refroidissement.

6. Procédé selon l'une quelconque des revendications 3 à 5, **caractérisé en ce que** le diamètre de l'étape de refroidissement Ca3) est inférieur au diamètre des étapes de refroidissement Ca1) et Ca2).

7. Procédé selon l'une quelconque des revendications 3 à 6, **caractérisé en ce que** la première étape de refroidissement Ca1) est configurée sous forme parallèle et doublement réversible.

8. Procédé selon l'une quelconque des revendications 1 à 7, **caractérisé en ce que** le milieu réfrigérant est introduit dans les zones de refroidissement par une ou plusieurs buses.

9. Procédé selon la revendication 8, **caractérisé en ce qu'**un écoulement dans lequel le nombre de Reynolds Re des deux phases du milieu réfrigérant est d'au moins 100 est généré dans la ou les buses.

10. Procédé selon l'une quelconque des revendications 1 à 5, **caractérisé en ce que** l'apport de puissance spécifique au volume dans le milieu réfrigérant est d'au moins $10^3$ W/m$^3$.

11. Procédé selon l'une quelconque des revendications 1 à 10, **caractérisé en ce que** le coefficient de variation de chaque composant du milieu réfrigérant lors de l'entrée dans les zones de refroidissement est inférieur à 1.

12. Procédé selon l'une quelconque des revendications 1 à 11, **caractérisé en ce que** l'étape Cb) comprend au moins une étape de compression Cba) et au moins une étape de refroidissement Cbb).

13. Procédé selon la revendication 12, **caractérisé en ce que** le milieu réfrigérant de l'étape de refroidissement Cbb) contient le même solvant organique que celui utilisé dans l'étape Ca) en tant que phase organique du milieu réfrigérant.

14. Procédé selon l'une quelconque des revendications 1 à 13, **caractérisé en ce que** l'étape Cb) comprend plusieurs étapes de compression Cba1) à Cban) et plusieurs étapes de refroidissement Cbb1) à Cbbn).

15. Procédé selon l'une quelconque des revendications 1 à 14, **caractérisé en ce que** l'étape D) comprend les étapes Da) à Dc) :

> Da) l'absorption des hydrocarbures en $C_4$ comprenant le butadiène et les n-butènes dans un agent d'absorption de point d'ébullition élevé, un courant d'agent d'absorption chargé avec des hydrocarbures en $C_4$ et le courant gazeux d2 étant obtenus,
> Db) l'élimination de l'oxygène du courant d'agent d'absorption chargé avec des hydrocarbures en $C_4$ de l'étape Da) par extraction avec un courant gazeux non condensable, et
> Dc) la désorption des hydrocarbures en $C_4$ du courant d'agent d'absorption chargé, un courant gazeux de produits en $C_4$ d1 étant obtenu, qui comprend moins de 100 ppm d'oxygène.

16. Procédé selon la revendication 15, **caractérisé en ce que** l'agent d'absorption de point d'ébullition élevé utilisé à l'étape Da) est un solvant hydrocarboné aromatique.

# FIG.1

# FIG.2

# FIG.3

# FIG.4

## IN DER BESCHREIBUNG AUFGEFÜHRTE DOKUMENTE

### In der Beschreibung aufgeführte Patentdokumente

- US 20120130137 A1
- US 20120130137 A
- JP 2011006381 A
- JP 2011001341 A
- JP 2013119530 A
- JP 2013177380 A
- WO 2012157495 A
- KR 20130036467
- KR 20130036468
- US 2014200381 A1
- WO 2014111406 A1
- US 4547615 A
- US 4424141 A
- DE 2530959 A
- US 3911039 A
- DE 2447825 A
- US 4423281 A
- US 4336409 A
- DE 2600128 A
- DE 2440329 A

### In der Beschreibung aufgeführte Nicht-Patentliteratur

- **JUNG et al.** *Catal. Surv. Asia,* 2009, vol. 13, 78-93
- *Applied Catalysis A: General,* 2007, vol. 317, 244-249
- Continuous Mixing of Fluids'' in Ull-mann's. **KRAUME et al.** Encyclopedia of Industrial Chemistry. Wiley-VCH, 2012
- **STREIFF.** *Chem. Ing. Tech.,* 1980, vol. 52, 520
- **MUSCHELKNAUTZ.** *Chem. Ing. Tech.,* 1979, vol. 51, 347
- *Erdöl und Kohle - Erdgas - Petrochemie,* vol. 34 (8), 343-346